# EUROPEAN PATENT APPLICATION

(11) **EP 4 803 057 A1**
(43) Date of publication of application: **09.09.2026**
(21) Application number: 25162296.5
(22) Date of filing: 07.03.2025
(51) Int. Cl.: A61F 13/532, A61F 13/533, A61F 13/536, A61F 13/539, A61F 13/551

(54) **ABSORBENT ARTICLE WITH ASYMMETRIC CHANNEL BOND**

(71) Applicant: The Procter & Gamble Company, Cincinnati, OH 45202 (US)
(72) Inventor: Garcia, Julien René, 65824 Schwalbach am Taunus (DE); Hu, Nan, 65824 Schwalbach am Taunus (DE); Peri, Andrea, 65824 Schwalbach am Taunus (DE); Shakaroun, Hossein, 65824 Schwalbach am Tauns (DE)
(74) Representative: P&G Patent Germany

(57) **Abstract**

An absorbent article (20) comprising an absorbent core (28) having a first channel and a second channel (26), each channel comprising a channel bond (27) between the core wrap top layer (45) and the core wrap bottom layer (46) characterized in that the channel bond (27, Wb) is asymmetric relative to the channel width (Wc) for at least a portion (68) of the first and second channels (26), so that, during use, the channel width preferentially shrinks on a pre-determined half (63) of the channel thus increasing the volume available for the absorbent material to swell in that pre-determined half (63) of the channel (26).

## Description

### FIELD OF THE INVENTION

The invention relates to personal hygiene absorbent articles such as diapers that comprise channels in their absorbent core.

### BACKGROUND OF THE INVENTION

Modern personal hygiene absorbent articles such as baby diapers and adult incontinence diapers are manufactured by converting multiple layers and components at high speed. Such absorbent articles comprise a fluid-permeable topsheet on the wearer-facing side of the article, a fluid-impermeable backsheet on the garment-facing side of the article, and an absorbent core disposed between these two layers. The absorbent core typically comprises an absorbent material disposed in a core wrap. The absorbent material has typically been a mixture of cellulose fibers and superabsorbent polymer particles ("SAP"). Over the years, the SAP content has increased relative to the cellulose fibers, thus providing thinner absorbent cores. Absorbent cores without cellulose fibers, so called airfelt-free cores, have also been recently proposed. Other typical components of diapers include an acquisition layer between the topsheet and the absorbent core, as well as inner and outer leg cuffs. Some absorbent articles comprise a dual layer acquisition-distribution system, especially in combination with airfelt-free absorbent cores.

Many baby diapers now comprise one or more elongated absorbent material-free channels in the absorbent core. These channels can help quickly distributing urine along their length to better use the absorbent capacity of the absorbent core. The top layer of the core wrap is typically bonded to the bottom layer of the core wrap in the channels to make the channels more durable against the swelling pressure of the swollen neighboring superabsorbent material. Semi-permanent channel bonds that delaminate when the absorbent core absorbs higher amount of fluid have been proposed. Exemplary disclosures of core with permanent or semi-permanent channels can be found in WO2012/170778 (Rosati et al.) and US2012/0312491 (Jackels).

Various improvement to channel designs have been proposed in the patent literature. For example WO2019/83711A1 (Bianchi et al.) discloses an absorbent article comprising at least two types of discrete channels within the absorbent material layer. A first type of channel is provided where the top side and bottom side of the core wrap are bonded together, and a second type of channel where the top and bottom sides are not bonded or significantly less bonded than in the first type. The channel bonding can be achieved through adhesive bonding, thermo bonding, mechanical bonding, ultrasonic bonding, or a combination of these methods. The first type of channels has a higher Static Peel Time compared to the second type.

Typically, the channel bonds have the same outline but are slightly smaller than the absorbent material-free areas in which they are formed due to the tolerance required in some manufacturing processes.

Channels in the absorbent core can also improve the flexibility of the diaper, reduce wet sagging and improve liquid distribution. However, at certain load, the channel bonds can constrain the core, making fluid absorption slower especially in the crotch where the SAP is more concentrated and most utilized. The effective capacity of the absorbent core can be lowered and also the kinetics of absorption may be reduced. While semi-permanent channels are thus designed to delaminate after a while when the diaper gets too full, if the channels open too early, the benefits of distribution and sustained fit could be lost. On the other hand, if the channel bonds open too late or are permanent, they may negatively impact the performance of the core when fully loaded.

The present invention aims at further improving the benefits of channels in an absorbent article.

### SUMMARY OF THE INVENTION

The present invention related in a first aspect to an absorbent article as indicated in the claims. The absorbent article comprises a front waist region, a crotch region, a back waist region, a longitudinal axis extending in a longitudinal direction, and a length L measured along the longitudinal axis. The crotch region is centered on the middle of the longitudinal axis and extends longitudinally over 50% of the length of the article. The article comprises a topsheet, a backsheet and an absorbent core between the topsheet and the backsheet. The absorbent core comprises a core wrap top layer, a core wrap bottom layer, an absorbent material layer between the core wrap top layer and bottom layer. The absorbent material comprises or consists of superabsorbent particles optionally mixed with cellulose fibers. The absorbent layer comprises a front edge, a back edge and two longitudinal edges. The absorbent material layer further comprises one side of the longitudinal axis a first longitudinally-elongated, absorbent material-free channel, and on the other side a second longitudinally-elongated, absorbent material-free channel, wherein the first and second channels are symmetrically disposed on each side of the absorbent layer relative to the longitudinal axis. The first and second channels may be connected to another or alternatively may be unconnected to another. The first and second channels are at least partially present in the crotch region of the article.

Each channel has an inner edge closer to the longitudinal axis, an outer edge closer to a longitudinal edge of the absorbent layer, a channel centerline disposed at equal distance from the inner edge and the outer edge, an outer half between the channel centerline and the outer edge, an inner half between the channel centerline and the inner edge, and a width as measured between the inner edge and the outer edge, wherein the width is constant or vary along the length of the channel.

The absorbent layer comprises a central absorbent area between the inner edges of the first and second channels, a first lateral absorbent area between the outer edge of the first channel and the closest longitudinal edge, and a second lateral absorbent area between the outer edge of the second channel and the closest longitudinal edge of the absorbent material layer.

The first channel and second channel each comprises a channel bond between the core wrap top layer and the core wrap bottom layer. The absorbent article is characterized in that in at least a portion of the first and second channels, the channel bond is asymmetric relative to the channel width, so that during use the channel width preferentially shrinks on a pre-determined half of the channel thus increasing the volume available for the absorbent material to swell in that pre-determined half of the channel. The bond strength on each channel half may be measured as the Static Peel Time in minutes for a section of the channel according to the Static Peel Time Test disclosed herein.

While in the prior art, channels are typically bonded with adhesive or ultrasonic or fusion bonds symmetrically disposed in the center of the channels, meaning there is no preferential delamination of the core wrap from one edge of the other, the asymmetric bond of the invention means that one side of the channel is having a higher bond strength compared to the other side of channel.

Various preferred features of the present invention are indicated in the following description and claims.

In a first feature, the channel bond in each of the first and second channels may be stronger adjacent the outer edge of the channel than the inner edge of the channel, so that the inner core wrap and outer core wrap preferentially delaminate first at the inner edge of the channel.

In a second feature, that can be combined with the first feature, at least a portion of the channels comprising the asymmetric bond may be substantially straight and parallel with the longitudinal direction. This portion of the channel may have a length of at least 5 cm. By "substantially parallel with the longitudinal axis", it is meant that the channel are straight and parallel with the longitudinal axis including a slight deviation of the channel centerline of d = 2 mm from the longitudinal axis direction.

In a third feature, that can be combined with the first feature and/or second feature, the potential swelling pressure in the central absorbent area may be higher than the potential swelling pressure in the lateral absorbent areas. The potential swelling pressure in each of the area is estimated by calculating the SAP/volume ratio in each of the absorbent area, the SAP/volume ratio being at least 20% higher in the central absorbent area than in each of the lateral absorbent area. The SAP/volume ratio is calculated by dividing the amount of SAP for each of the absorbent area and dividing this amount by the square of the width of the absorbent area considered.

These features are beneficial as the channel can widthwise shrink from the side where the SAP swelling is most constrained. Typically, the SAP is more utilized in the central absorbent area due to gravity during usage so that the core performance is maximized in the center of the core.

In another feature, the channel bond is at least partially formed by an auxiliary glue present between the absorbent material layer and at least one of the core wrap top layer and the core wrap bottom layer on which the auxiliary glue layer was applied. The auxiliary glue preferably comprises a plurality of parallel and longitudinally oriented auxiliary glue stripes, with two adjacent stripes being separated by a gap. The auxiliary glue stripes may comprise an irregular pattern. By "irregular", it is mean that the auxiliary glue stripes are not all identical but that at least two stripes and/or two gaps between adjacent stripes are different. In particular, the auxiliary glue stripes may comprise at least two parallel stripes having different widths and/or adjacent stripes having at least two different gaps between them. Due to the irregular pattern of the auxiliary glue stripes, the amount of glue deposited varies perpendicularly to the glue stripes. In this way, it is possible to apply glue closer to the inner edge or the outer edge of the channel, as desired. This is especially practical when the first and second channel, or at least a substantial portion thereof, are substantially parallel with the longitudinal axis of the article.

The first and second channels may be substantially parallel with the longitudinal axis of the article over their entire length. Alternatively, the channels may be parallel with the longitudinal axis at least in their longitudinal middle portion and curved towards a front and/or back portion. Having a curved portion of the channel at one or both its extremities together with an irregular auxiliary glue pattern may in particular be useful to vary the bond strength of the channel along the length of the channel. The channel may comprise a front portion and a back portion each having a higher bond strength than in a channel middle portion disposed between the front portion and the crotch portion. Thus the bond strength of the channel may be varied along the length of the channel so that the channel delaminates more or less easily in pre-determined longitudinal portions of the channel.

### BRIEF DESCRIPTION OF THE DRAWINGS

Fig. 1 is a top view of the wearer-facing side of an exemplary article of the invention in the form of a taped diaper which has been pulled flat, with some layers partially removed;
Fig. 2 shows a cross-section view of the taped diaper of Fig. 1;
Fig. 3 shows an exploded view of the taped diaper of Fig. 1;
Fig. 4 shows a perspective view of a diaper pant;
Fig. 5 shows a top view of the wearer-facing side of the diaper pant;
Fig. 6 shows a top view of an exemplary absorbent core that may be used in one of the articles of Figs 1-5, with the top side of the core wrap partially removed;
Fig. 7 shows a schematic view of the absorbent core in exploded view;
Fig. 8 shows the irregular adhesive pattern used to provide the asymmetric bond in one of the channel of the core of Fig. 7;
Fig. 9 shows a cross-section of the core before use in a channel area along line 9-9 in Fig. 8;
Fig. 10 shows the same cross-section as Fig- 9 in the wet state, wherein the asymmetric channel delaminated on the inner edge of the channel to give room for the absorbent material in the central absorbent area to swell;
Fig. 11a,b,c show closeup views of the auxiliary glue adhesive pattern in different portions of the channel;
Fig. 12 shows a simplified view of the channel of Fig. 8, showing the portion 68 of the channel that is straight and longitudinally oriented (d= 2 mm);
Figs. 13-17 illustrate how to conduct the Static Peel Time Test, further described hereinbelow.

### DETAILED DESCRIPTION OF THE INVENTION

### Absorbent article

As used herein, "absorbent articles" refers to personal hygiene devices that are designed to be placed in contact to the body of a wearer to absorb and contain body exudates. The absorbent articles of the invention include baby care articles, feminine care articles or adult incontinence articles.

Baby care articles are products intended for babies, toddlers and/or children, including taped diapers, pant diapers, absorbent inserts, infant and/or toddler care wipes, and infant and/or toddler bibs.

Feminine care articles are products relating to catamenial pads, incontinence pads, interlabial pads, panty liners, pessaries, sanitary napkins, tampons and tampon applicators, and/or wipes.

Adult incontinence articles are products intended for adults, relating to disposable absorbent articles including taped diapers, pant diapers, absorbent inserts, incontinence pads, panty liners, and vaginal inserts.

The absorbent articles of the invention are typically disposable and are preferably recyclable.

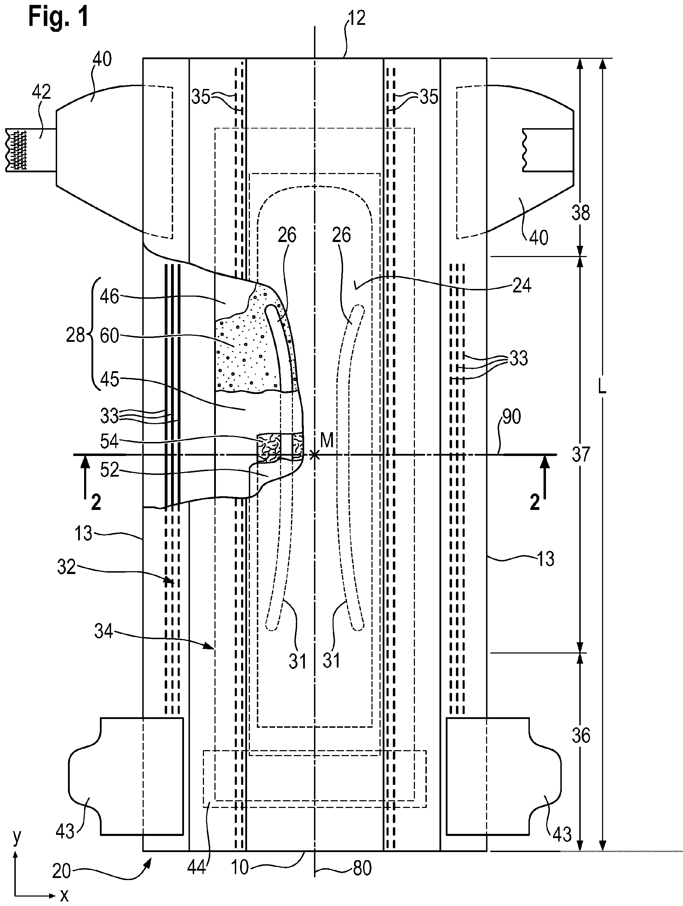

As used herein, "diapers" refers to absorbent articles designed to be worn by babies, infants or incontinent adults about the lower torso, so as to encircle the waist and legs of the wearer, and that are specifically adapted to receive and contain urinary and fecal waste. Diapers are typically proposed as taped diapers or pant diapers. Taped diapers have a fastening system (as illustrated in Fig. 1 for example), where the waist opening and leg openings are formed when the diaper is applied onto the wearer by releasably attaching the longitudinal edges of the front waist region and back waist region to each other. In pant diapers, on the other hand the longitudinal edges of the waist regions are attached to each other to form a pre-formed waist opening and leg openings. A pant diaper is placed in position on the wearer by inserting the wearer's legs into the leg openings and sliding the pant diaper into position about the wearer's lower torso. A pant may be pre-formed by any suitable techniques including, but not limited to, joining together portions of the absorbent article using re-fastenable and/or non-refastenable bonds (e.g., seam, weld, adhesive, cohesive bond, fastener, etc.). A pant may be pre-formed anywhere along the circumference of the article (e.g., side fastened, front waist fastened).

### General description of an absorbent article 20, 20'

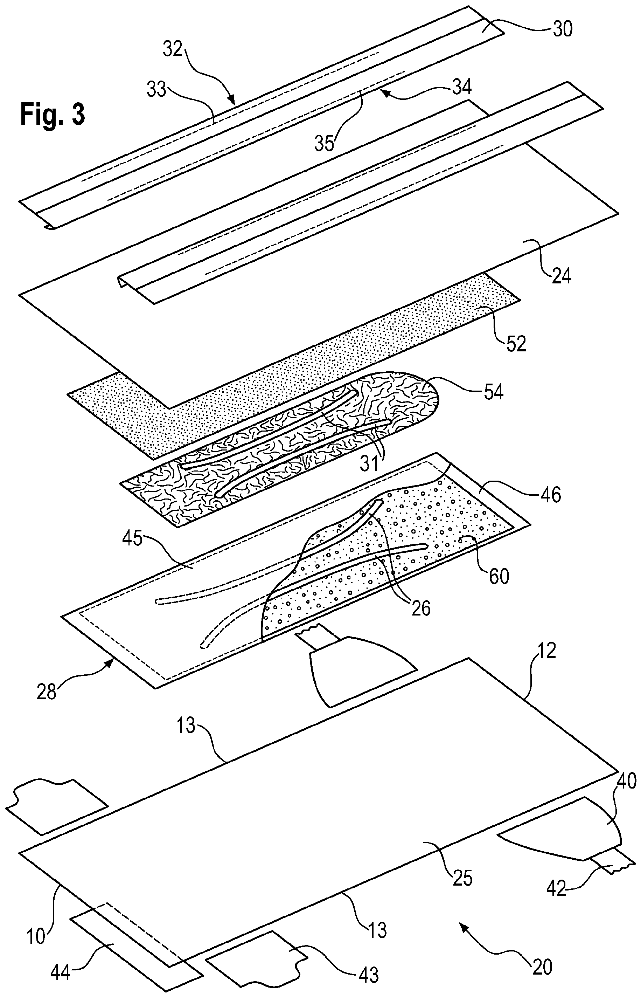

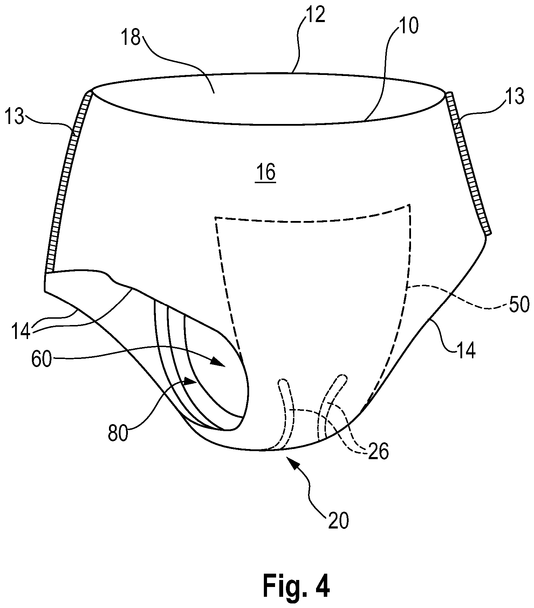

A generic description of an absorbent article in the form of a diaper is discussed herein with reference to the exemplary, non-limiting absorbent articles shown in Figures 1-3 for a taped diaper 20 and in Figs. 4.5 for a pant diaper. Figure 1 is a plan view of such an exemplary diaper 20, in a flat-out state, with portions of the diaper being cut-away showing the different layers of the diaper. This diaper 20 is shown for illustration purpose only, as the present invention may be comprised in a wide variety of diapers or other absorbent articles, in particular in pant diapers having pre-formed side seams, as illustrated in Fig. 4. The side seams of pant articles can be opened by cutting or otherwise, if it is desired to place the pant in a flattened-out configuration similar to Fig. 1.

As illustrated in Fig. 1, the absorbent article 20 comprises a front edge 10, a back edge 12, and two longitudinally-extending side (lateral) edges 13. The front edge 10 is the edge of the article which is intended to be placed towards the front of the user when worn, and the back edge 12 is the opposite edge. The absorbent article may be notionally divided by a longitudinal axis 80 extending along a longitudinal direction y from the middle of the front edge 10 to the middle of the back edge 12 of the article. The longitudinal axis thus notionally divides the article in two substantially symmetrical left and right halves relative to this axis, when viewing the article from the wearer-facing side in a flat-out configuration, as exemplarily shown in Fig. 1.

The article has a length L as measured from its front end to its back end along the longitudinal axis. The transversal centerline 90 is the imaginary line perpendicular to the longitudinal axis 80 in the plane of the flattened-out diaper and notionally dividing the article into a front half and back half having an equal length of L/2.

If some parts of the article are under tension due to elasticized components, the article may be typically flattened using clamps along the periphery of the article and/or a sticky surface, so that the article can be pulled taut to be substantially flat. Closed articles such as pant-like baby diapers, training pants for small children, or adult incontinent pants may be cut open along the side seams to apply them on a flat surface, as is known in the art. Unless otherwise indicated, dimensions and areas disclosed herein apply to the article in this flat-out configuration.

The absorbent article, whether a taped diaper or a pant diaper, can be notionally divided in a front waist region 36, a back waist region 38 opposed to the front waist region 36, and a crotch region 37 located between the front waist region 36 and the back waist region 38. The crotch region is defined as being the middle 50% of the article, the front waist region and the back waist region are being the remaining 25% towards the front and back of the article respectively, as measured along the longitudinal axis 80.

As illustrated in Figures 1-3, the absorbent article comprises a topsheet 24 on its wearer-facing side, a backsheet 25 on its garment-facing side, and an absorbent core 28 between the topsheet 24 and the backsheet 25. The absorbent core 28 comprises at least one absorbent layer 60 comprising superabsorbent polymer particles ("SAP") and a core wrap. The absorbent layer 60 is disposed between a top core wrap layer 45 and a bottom core wrap layer 46. The absorbent core also comprises a longitudinal centerline 80', which in an article is generally aligned with the longitudinal centerline 80 of the article, subject to manufacturing tolerance. In the following when discussing the article, reference is made to the longitudinal centerline 80 of the article, and when discussing the core separately, reference is made to the longitudinal centerline 80' of the core.

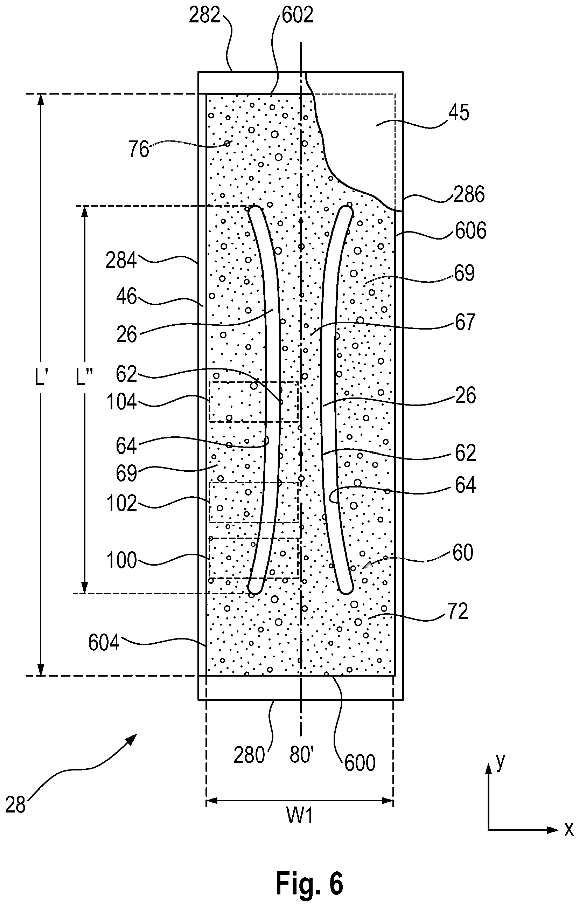

The absorbent layer 60 typically has a pre-determined outline (periphery) as considered in the plane formed by the article when flattened out. This outline may be substantially rectangular as shown on Fig. 6, but the absorbent layer may also have a shaped periphery such as a sand-hour or dog-bone shape. The absorbent core 28 further comprises a first elongated, absorbent material-free channel 26, which is an area substantially free of absorbent material within the absorbent layer 60, as well as a second absorbent material-free channel 26, which symmetrically disposed on the other side of the longitudinal axis 80'. The first channel may also be designated as the left channel, and the second channel as the right channel, when considering the absorbent core or the absorbent article from the top as shown in Fig. 1 and Fig. 6. Since the first and second channels are generally similar the same reference number 26 is used for these interchangeably, and in this description they may be referred in the singular or plural form. The channel is typically elongated in the longitudinal direction, so that it extends more, preferably at least 4 time more in the longitudinal direction y than in the transversal direction x. The channels 26 facilitates the distribution of urine along the length of the absorbent article.

The absorbent articles of the invention may further comprise an acquisition-distribution system between the topsheet and the absorbent core. The acquisition-distribution system may be comprised of a single acquisition layer, or alternatively as represented it may comprise an upper acquisition layer 52 directly underneath the topsheet, and a distribution layer 54 between the acquisition layer and the absorbent core.

An example of acquisition layer 52, which may be used alone or with a distribution layer, may be a surfactant treated, latex bonded, nonwoven acquisition layer. The distribution layer 54 may be a layer of cross-linked cellulose fibers, especially when the absorbent core 28 is an airfelt-free absorbent core. Another example of acquisition layer 52 is a spunlace layer, which may be used alone or in combination with a distribution layer 54.

Nonwovens are sheet or web structures bonded together by entangling fibers or filaments mechanically, thermally, or chemically. They are flat, porous sheets that are made directly from separate fibers. They are not made by weaving or knitting and do not require converting the fibers to yarn. Common process to make nonwovens are meltblowing, spunbonding, solvent spinning, electrospinning, carding and airlaying. The basis weight of nonwoven webs is usually expressed in grams per square meter (g/m2 or gsm).

Suitable spunlace nonwovens comprise absorbent fibers, stiffening fibers and resilient fibers, as for example disclosed in WO2020/205485 (Peri et al.). The spunlace nonwoven may typically comprise from about 20 percent to about 75 percent of absorbent fibers, from about 1 percent to about 50 percent of stiffening fibers, and from about 10 percent to about 50 percent of resilient fibers. However other materials having fluid acquisition and distribution properties and integrity may be used.

Further, the absorbent article may comprise other optional but conventional elements, which are not represented for simplicity, such as a back waist elastic feature, a front waist elastic feature, a lotion applied onto the body-facing surface of the topsheet, or a urine indicator disposed on the inner side of the backsheet that changes color when contacted with urine.

The topsheet 24, the backsheet 25, and the absorbent core 28 may be assembled in a variety of well-known configurations, such as by gluing, heat embossing, ultrasonic bonding or combinations thereof. Exemplary diaper configurations are described generally in US3,860,003; US5,221,274; US5,554,145; US5,569,234; US5,580,411; and US6,004,306.

### General description of a pant diaper 20'

Pant diapers can be generally similarly constructed as taped diaper as discussed above with the following differences. Fig. 4 depicts an example of a belt- or balloon-type disposable absorbent pant 20'. The pant 20' has a waist opening defined by a front waist edge 10' and rear waist edge 12' and a pair of leg openings defined by respective leg opening edges 14. The pant 10 includes a belt structure having a front panel 16 and a rear panel 18, which are joined at side seam areas 13. Typically, side seams are formed by welding/thermal bonding in which polymer materials in the front and rear panels are fused together at their edges by application of a combination of heat and pressure. The pant 20' includes an absorbent pad assembly 50 extending between the front and back panels through a crotch region of the pant. The absorbent pad assembly comprises the topsheet, backsheet and the absorbent core, and typically other components such as acquisition layer and barrier cuffs. The absorbent pad assembly 50 may be adhesively bonded to the inner surfaces of front and rear panels, typically a hotmelt adhesive is applied during the manufacturing process for this purpose. The front belt 16 and the back belt 18 typically comprise an elasticized laminate, such as a plurality of elastic threads 22 extending in the transversal direction and sandwiched between two nonwoven substrates.

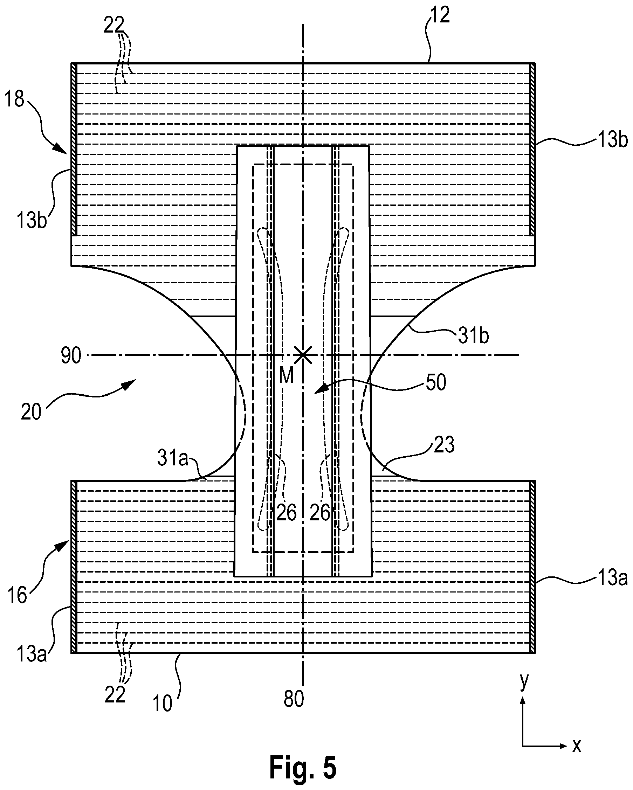

Fig. 5 shows the pant article of Fig. 4 with the side seams 13a,b opened so that the article can be shown flattened out with the wearer-facing side of the pant shown. As shown in Fig. 5, the article extends in a longitudinal direction y parallel to the longitudinal axis 80 and a transversal direction x parallel to the transversal centerline 90. The longitudinal axis 80 extends from the middle of the front edge 10 of the front belt 16 to the middle of the back edge 12 of the rear belt 18, and notionally divides the article in a left half and a right half, which are substantially symmetrical. The transversal centerline 90 is disposed in the crotch portion of the article and divides the article in a front half and a rear half having an equal length as measured along the longitudinal centerline 80. The intersection of the longitudinal centerline 80 and transversal centerline 90 is defined herein as the midpoint M, as for a taped diaper.

Referring further to Fig. 5, the pant 20 includes a belt structure including front panel 16 and rear panel 18. In the example depicted, the outer layers of the front and rear panels comprise a continuous layer 23 common to both panels. The continuous layer 23 also wraps about the outside of the pant through the crotch region; this configuration is sometimes called a "unibody" construction. The outer layer(s) 32 is typically a nonwoven layer. In another possible configuration (not shown), front panel 16 and rear panel 18 may have discrete, separate outer layers, have no layer in common, and are joined only by the side seams 13 and the absorbent pad assembly. This alternative configuration is sometimes called a "multipiece" construction.

Each of the front and rear panels may comprise one or more elastic members, in particular a plurality of transversally extending strands 22 of an elastomeric material laminated between an inner belt layer (not shown) and an outer belt layer 32, such as elastane threads (for example, LYCRA HYFIT fiber, a product of Invista, Wichita, Kansas). The inner and outer belt layers may respectively be joined together about the elastic strands 22 by adhesive deposited between the layers, by thermal bonds, by compression bonds, or by a combination thereof. In other examples, the one or more elastic members may be strips or a section of film formed of elastomeric material. Elastic strands rather than film, however, may be preferred for the flexibility they provide by enabling individualized setting of pre-strain levels, selecting and setting uniform or varying longitudinal spacing therebetween, and preserving a high level of vapor transmission (breathability) through the belt laminate, for purposes of coolness, comfort and skin health. This flexibility helps enable the manufacturer to enhance the fit of the pant structure about the varying contours and sizes of differing wearers' anatomies, and impart a cloth-like appearance to the belt laminate. For purposes herein, a "strand" is a member having a cross-section perpendicular to its longest dimension, the cross-section having an aspect ratio of largest dimension to smallest dimension no greater than 2, in an unstrained condition.

The elastic belt may also or alternatively comprise an elastomeric nonwoven. Further typical features of an incontinence pant article, in particular the belt/panel structure can be found for example in WO2018/09417A1 (Minoguchi et al.), WO2017/192992A1 (Desai et al.), and WO2016/115421A1 (Seitz et al.).

Having generally described two examples of absorbent articles in the form of a taped diaper and a pant diaper, exemplary components thereof will now be further detailed individually.

### Topsheet

The topsheet 24 forms the wearer-facing surface of the article that is in intimate contact with the skin of the wearer, in particular the perineal area. At least a portion of and typically all the topsheet is liquid permeable, permitting liquid bodily exudates to readily penetrate through its thickness. The topsheet may consist or comprise a carded calendered nonwoven according to the invention, benefitting from the excellent softness of the nonwoven of the invention, especially when the staple fibers comprise a combination of natural and synthetic fibers.

The topsheet may have one or more layers. The topsheet may be apertured or non-apertured, and may have any suitable three-dimensional features, and/or may have a plurality of embossments (e.g., a bond pattern). Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet.

### Backsheet

The backsheet 25 is generally the portion of the absorbent article 20 that constitutes all or a part of the garment-facing surface of the absorbent article. The backsheet 25 may be joined at least partially to the topsheet 24, the absorbent core 28, or other layers of the article by any attachment methods known to those of skill in the art. The backsheet prevents, or at least inhibits, the bodily exudates absorbed and contained in the absorbent core from soiling articles such as bedsheets, undergarments, and/or clothing. The backsheet is typically liquid impermeable, or at least substantially liquid impermeable.

The backsheet typically comprises a thin impermeable plastic film, usually a thermoplastic film having a thickness of about 0.01 mm to about 0.05 mm. The backsheet material may be breathable, which permit vapors to escape from the absorbent article, while still preventing, or at least inhibiting, bodily exudates from passing through the backsheet. A breathable backsheet may have a Water Vapor Transmission Rate (WVTR) of from 1,000 to 15,000 g/m²/24h, or from 1,000 to 10,000 g/m²/24h, or from 1,500 to 10,000 g/m²/24h as measured using a PERMATRAN-W Model 101K (available from Mocon, Inc., Minneapolis, MN) or equivalent, according to Nonwovens Standard Procedure NWSP 70.4.R0(15) with the following specifications: experiments were carried out in a lab controlled at 23 °C ± 2 C° and 50 %RH ± 2 %RH and the instrument cells heated to 37.8°C (100°F).

The backsheet 25 may also comprise a backsheet outer cover nonwoven (not represented separately in the Figures). The backsheet outer cover nonwoven is typically a thin nonwoven material that is joined to the outer surface of the backsheet film. The outer cover nonwoven may thus form the garment-facing surface of the backsheet. The backsheet may thus comprise a carded calendered nonwoven according to the invention. Especially the nonwoven of the invention may form the backsheet outer cover, profiting from the improved softness properties of the nonwovens of the invention. The backsheet outer cover nonwoven may also comprise a bond pattern, apertures, and/or three-dimensional features, and may improve the feel of the backsheet.

### Barrier cuffs 32, 34

The absorbent article 20 may also comprise inner barrier leg cuffs 34 and outer leg cuffs 32, as is known in the art. The inner barrier cuffs 34 can extend upwards from the surface of the article to provide retention of the waste, while the outer cuffs 32 are typically formed in the plane of the chassis of the article as defined by topsheet and backsheet. These cuffs are preferably elasticized, as is known in the art, for example using elastic threads 33, 35 as represented in the Figures 1-2. In a pant diaper comprising an inner and outer belt nonwovens as is known in the art, the barrier cuffs are typically part of the absorbent pad chassis 50.

Moreover, the absorbent article may comprise a fastening system, such as an adhesive fastening system or a hook and loop fastening member, which can comprise tape tabs 42 disposed on back ears 40, such as adhesive tape tabs or tape tabs comprising hook elements, cooperating with a landing zone 44 (e.g. a nonwoven web providing loops in a hook and loop fastening system). While taped diapers typically comprise back ears 40, and front ears 43, these are typically not present in pant-type absorbent articles having pre-formed side seams.

The front and/or back ears may be separate components attached to the absorbent article or may instead be continuous with portions of the topsheet and/or backsheet such that these portions form all or a part of the front and/or back ears 40, 43. Also combinations of the aforementioned are possible, such that the front ears 43 and/or back ears 40 are formed by portions of the topsheet and/or backsheet while additional materials are attached to form the overall front and/or back ears 40, 43. The front and/or back ears may be elastic or non-elastic. Also, the front ears 40 may be applied as separate components attached to the absorbent article while the back ears (or parts thereof) may be continuous with portions of the backsheet and/or topsheet - or vice versa.

### Liquid management layers 52, 54

The absorbent article may advantageously comprise at least one liquid management layer 52, also referred herein as acquisition layer and/or distribution layer, between the topsheet 24 and the absorbent core 28 as illustrated in Figs. 1-2. Liquid management layers quickly acquire and/or distribute the fluid away from the topsheet and into the core. These liquid management layers are sometimes called "wicking layer", "surge layer", "acquisition layer", "distribution layer" or "acquisition-distribution layer". Typically, liquid management layers do not comprise SAP, as this may slow the acquisition and distribution of the fluid. The prior art discloses many type of liquid management layer, see for example WO2000/59430 (Daley), WO95/10996 (Richards), US5,700,254 (McDowall), WO02/067809 (Graef). Liquid management layers are typically placed symmetrically relative to the longitudinal centerline of the article.

The liquid management layer 52 may be made of a nonwoven web having hydrophilic properties, often referred to as an acquisition layer. The nonwoven web may be for example provided as a continuous roll of material that is cut according to the desired length and pattern as it is unwound in a converting line. Such an acquisition layer is typically placed directly under the topsheet. Suitable nonwovens are for example through-air bonded ("TAB") carded nonwovens, resin-bonded ("RB") carded nonwovens, spunbond or spunlace (hydroentangled) nonwovens. TAB carded nonwovens may for example be made from soft PE/PP bicomponent staple fibers. The air through bonding process locks in loft and compressibility. Resin-bonded carded nonwovens may be made from multi-denier polyester staple fibers (for example: 50/50 or 40/60 mix of 6 denier and 9 denier fibers). Its resilient and open structures are designed to provide excellent fluid acquisition properties. Such acquisition layers are available directly from suppliers, e.g. Fitesa of Simpsonville, South Carolina, USA or TWE Group GmbH, of Emsdetten, Germany. The nonwoven layer may be stabilized by a latex binder for example a styrene-butadiene latex binder (SB latex). Processes for obtaining such latexes are known, for example from EP149,880 (Kwok), US2002/028858 and US2003/0105190 (Diehl). The binder may typically be present in an acquisition layer in excess of about 12%, about 14% or about 16% by weight of the layer. A SB latex is for example commercially available under the trade name GENFLO^{™} 3160 (OMNOVA Solutions Inc.; Akron, Ohio). Latex bonded acquisition layers are for example further disclosed in US2005/033252A1, US2005/033253A1 or US2005/043694A1 (Schneider). The basis weight of typical acquisition layers ranges from 10 gsm to 200 gsm, in particular 20 gsm to 140 gsm, or 40 gsm to 120 gsm, for example 80 gsm.

Since the function of a liquid management layer is to help transfer the insulting fluid to the absorbent material, it is generally not desirable for the liquid management to extend beyond the edges of the absorbent material area of the core. The liquid management layer may be typically rectangular, and its width may be shorter in the transversal direction than the minimum width of the absorbent material layer of the absorbent core.

When the absorbent material comprises, cellulose fibers mixed with the SAP, a single liquid management layer 52 in the form a hydrophilic nonwoven acquisition layer may be sufficient. Cellulose fibers can typically help acquiring and distributing the fluid within the core. Where the absorbent material of the core is substantially free of cellulose fibers however, it may be advantageous to have two liquid management layers, in the form of the combination of an acquisition layer 52 and a distribution layer 54, as described below and illustrated in any of Figs. 1-3.

The absorbent articles of the invention may comprise a first and a second liquid management layers. These layers may form an integrated unitary layer or remain discrete layers which are attached to each other for example by adhesive bonding. The article may comprise an acquisition layer 52 directly under the topsheet and a distribution layer 54 between the acquisition layer and the absorbent core, as illustrated in any of Figs. 1-3. The distribution layer 54 may comprise cross-linked cellulosic fibers and the acquisition layer comprised of a carded, resin-bonded hydrophilic nonwoven. The distribution layer may be profiled having a narrower crotch or have a generally rectangular. Distribution layer with a rounded end towards the back of the diaper ("bullet" shaped) have been suggested. Such dual layer liquid management layers are for example disclosed in further details in WO2014/093323 (Bianchi et al.). In the example shown in Fig. 1-3, the distribution layer 54 is bullet shaped with a fitted crotch portion have a narrower width relative to the front and back portion of the distribution layer.

Such a fibrous distribution layer 54 may for example be made on-line by depositing the fibers, for example cross-lined cellulosic fibers, on a forming surface having ridges corresponding to the areas where no fibrous material is desired. Deposition chambers are known wherein a carrier sheet is provided on a forming surface having a series of holes connected to a vacuum, so that the vacuum pulls the fibers in the desired emplacements to form a desired deposited layer. The forming surface of these deposition chambers can be modified to provide a layer of fibrous material having a central portion and side portions separated by folding guides. The fibrous layer is typically formed or transferred on a carrier sheet that should thus have at least the same dimension as a fibrous liquid management layer. The carrier sheet may be the topsheet, another liquid management layer such as a nonwoven acquisition layer 52, or any other layer of the article, for example the core wrap.

The function of a distribution layer is to spread the insulting fluid liquid over a larger surface within the article so that the absorbent capacity of the core can be more efficiently used. Typically, distribution layers can be made of a material comprising synthetic or cellulosic fibers and having a relatively low density. The distribution layer material may be a nonwoven or a fibrous layer comprising unbound or loosely bound hydrophilic fibers, in particular a layer of cross-linked cellulosic fibers. The density of the distribution layer may vary depending on the compression of the article but may typically range from 0.03 g/cm3 to 0.25 g/cm3, in particular from 0.05 g/cm3 to 0.15 g/cm3 measured at 0.30 psi (2.07kPa). The distribution layer may also be a material having a water retention value of from 25 to 60, preferably from 30 to 45, measured as indicated in the procedure disclosed in US5,137,537.

Such a distribution layer 54 may for example comprise at least 50% by weight, optionally consisting of 100%, of cross-linked cellulosic fibers. The cross-linked cellulosic fibers may be crimped, twisted, or curled, or a combination thereof including crimped, twisted, and curled. This type of material has been used in the past in disposable diapers as part of an acquisition system, for example US 2008/0312622 A1 (Hundorf), however not in the manner of the invention. The cross-linked cellulosic fibers provide higher resilience and therefore higher resistance against the compression in the product packaging or in use conditions, e.g. under baby weight. This provides the layer with a higher void volume, permeability and liquid absorption, and hence reduced leakage and improved dryness. The liquid management layer 54 may also be typically profiled so that more material is present at the front and middle part of the article relative to the back of the article. The distribution layer may typically have an average basis weight of from 30 g/m² to 400 g/m², in particular from 100 g/m²to 300 g/m², with the basis weight varying along the length of the article so that more material is present at the front and middle of the layer than at the back. The liquid management layer may thus be profiled and/or shaped rounded towards the back of the article, as exemplarily disclosed in WO2014/093323 (Bianchi).

The distribution layer 54 may be provided with folding guides 31 as illustrated in Figs. 1-3. The folding guides 31 may be at least partially vertically superposed with the channels 26 of the absorbent core, as illustrated in Figs. 1-3. Thus, the liquid management layer 54 can easily fold similarly to the core when adapting to a three-dimensional basin shape. "Vertically superposed" means that the position and shape of the folding guides of the liquid management layer vertically correspond to the underlying channel-forming areas of the absorbent core, so that the liquid management layer can readily assume a basin-like shape formed by the underlying absorbent core when the article is put on and worn by the wearer.

The folding guide(s) 31 of the liquid management layer may be superposed with the core channel(s) 26 of the absorbent core over the whole length of folding guides, but a lower percentage of overlap is also possible. For example, the liquid management layer's folding guides may overlap over at least 50%, 60%, 70% or more of the overall length of the absorbent core's channel-forming area(s). In the remaining areas where there is no overlap, the liquid management layer's folding guides may for example be off-set relative to the absorbent core's channel-forming area(s) or may be shorter and thus not extend to the same length as the absorbent core channel(s) 26.

If folding guide(s) 31 are present in such a distribution layer, they may be formed by areas substantially free of the liquid management material, in this case substantially free of unbound or loosely bound hydrophilic fibers such as cross-linked cellulosic fibers as illustrated in Figs. 1-3.

The invention is however not restricted to this example having two liquid management layers. Many disposable absorbent articles have in particular for cost reason only one liquid management layer 52. As indicated previously, there may also be no liquid management layer between the absorbent core and the topsheet, and/or one such layer may be present under the absorbent core, between the absorbent core and the backsheet.

### Absorbent core 28

The absorbent articles of the invention comprise an absorbent core between the topsheet and the backsheet. The absorbent core 28 comprises an absorbent layer 60 and a core wrap comprising a core wrap top layer 45 and a core wrap bottom layer 46. The absorbent layer comprises superabsorbent polymer particles (SAP) optionally mixed with cellulose fibers. SAP are water-insoluble, water-swellable polymers capable of absorbing large quantities of fluids, as is known in the art.

"Superabsorbent polymer" or "SAP" refers herein to absorbent materials, typically cross-linked polymeric materials, that can absorb at least 10 times their weight of an aqueous 0.9% saline solution as measured using the Centrifuge Retention Capacity (CRC) test as indicated in EDANA method NWSP 241.0.R2 (19). The SAP may in particular have a CRC value of at least 20 g/g, or more than 24 g/g, or of from 20 g/g to 50 g/g, or from 20 g/g to 40 g/g, or 24 g/g to 35 g/g. "Superabsorbent polymer particles", as used herein, refers to a superabsorbent polymer material which is in particulate form so as to be flowable in the dry state.

The absorbent articles of the invention comprise an absorbent core between the topsheet and the backsheet. The absorbent core 28 comprises an absorbent layer 60 and a core wrap comprising a core wrap top layer 45 and a core wrap bottom layer 46. The absorbent layer comprises superabsorbent polymer particles (SAP) optionally mixed with cellulose fibers and/or synthetic fibers. SAP are water-insoluble, water-swellable polymers capable of absorbing large quantities of fluids, as is known in the art.

The absorbent layer 60 is typically sandwiched, and preferably immobilized, between the core wrap top layer 45 and the core wrap bottom layer 46, so that the absorbent core can be easily integrated with the rest of the chassis of the absorbent article in a converting line.

Superabsorbent polymer particles can also be mixed with cellulose fibers (airfelt cores) or may be incorporated between the pores of synthetic fibers comprised in a high loft nonwoven. The absorbent core may alternatively comprise at least one layer of SAP, wherein the SAP particles are not mixed with cellulose fibers and/or synthetic fibers. The resulting layer of absorbent material may thus have a reduced thickness in the dry state, compared to conventional airfelt-based absorbent cores. The reduced thickness helps to improve the fit and comfort of the absorbent article for the wearer. The absorbent material may be free of cellulose fibers (however the absorbent core may still comprise some cellulose fibers in a nonwoven or tissue layer, such as a spunlace layer, for example as a core wrap layer). The superabsorbent

Various absorbent core designs comprising a layer of SAP free of cellulose fibers have been proposed in the past, see for example in US5,599,335 (Goldman), EP1,447,066 (Busam), WO95/11652 (Tanzer), US2008/0312622A1 (Hundorf), WO2012/052172 (Van Malderen). In particular, the SAP printing technology as disclosed in US2006/024433 (Blessing), US2008/0312617 and US2010/0051166A1 (both to Hundorf et al.) may be used. A method of making channels in such an airfelt-free core is disclosed in WO2012/170798A1 (Jackels et al.) wherein the receiving drum (lay-down drum) which supports the core wrap layer on which the SAP particles are deposited comprises longitudinally mating strips corresponding to the shape of the desired channels that mate with raised trips on the SAP printing drum. WO2015/1253088A1 (Armstrong-Ostle et al.) discloses an improvement to the process in which an auxiliary bonding drum is used to apply pressure by means of mating strips that corresponds to the mating strips on the lay-on drum thus providing for overall better channel bond strength between the adhesive and the core wrap layers in the channel. Similar processes for making the channel bonds but in the context of an airfelt (a mixture of cellulose fibers and SAP particles) absorbent material are known, for example as disclosed in WO2020/188047.

The layer of absorbent material may be typically deposited on at least one layer of the absorbent core serving as substrate, such as the bottom core wrap layer or top core wrap layer. In the SAP-printing process as described in US2008/312,622A1 (Hundorf), a substantially continuous layer of SAP is obtained by depositing SAP on each of the core wrap layers in a pattern having absorbent material land areas separated by absorbent material-free junction areas. The absorbent material land areas of the first layer correspond substantially to the absorbent material-free junction areas of the second layer and vice versa, so that a continuous layer of SAP is obtained when the two discontinuous layers are combined. Of course, the absorbent material-free channel is then formed by a matching absorbent material-free area on the first and second absorbent layers.

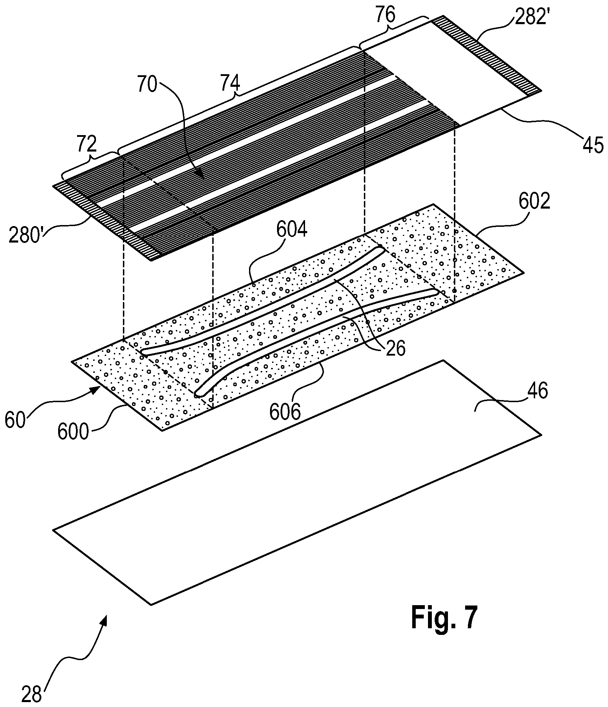

As illustrated in Fig. 7, the absorbent core comprises an auxiliary glue 70 disposed between the absorbent material 60 and at least one of the core wrap top layer 45 or bottom layer 46. The auxiliary glue at least partially immobilizes the absorbent material on said core wrap top layer or bottom layer by adhering at least some of the absorbent material to the substrate. The auxiliary glue may be disposed on only of the core wrap top layer or bottom layer or may be disposed on both layers. If the auxiliary glue is disposed on both layers, the auxiliary glue pattern may be the same on each layer or may be different. While the auxiliary glue is typically applied in the prior art in regular pattern, typically using a slot glue applicator comprising a shim having a regular slot interval to provide for a regular application of the auxiliary glue stripes, on one of core wrap top layer or bottom layer as substrate, in the present invention, the auxiliary glue is on the other hand be applied in an irregular pattern, as will be discussed further below.

A micro-fibrous thermoplastic adhesive net may also be used in airfelt-free cores as described in the above Hundorf references to immobilize SAP particles on at least one of the substrates formed by the top and bottom core wrap layers. These adhesives are not represented in the Figures for simplicity.

The basis weight (amount deposited per unit of surface) of the superabsorbent material may also be varied to create a profiled distribution of superabsorbent material, in particular in the longitudinal direction to provide more absorbency in the crotch portion of the article, but also in the transversal direction, or both directions of the core.

The core wrap is formed by one or two substrate layers that sandwiches the SAP particles and a least partially immobilizes the SAP particles so that the absorbent core keeps its integrity. The core wrap top layer 45 and the core wrap bottom layer 46 are also referred to in the art as core cover and dusting layer respectively. These core wrap layers are typically low basis weight nonwoven (typically less than 20 gsm, in particular from 8 gsm to 14 gsm), and may be in particular SMS nonwoven (Spunbond-Meltblown-Spunbond laminate), as is known in the art. The upper and lower core wrap layers may be any material capable of containing and providing a support for the absorbent material.

The core wrap layers may be made of the same or different materials, i.e. two nonwoven webs which have the same of different properties e.g. in a c-wrap configuration. The core wrap may also be made of a single, continuous nonwoven web, which is wrapped around the layer of absorbent material as this may simplify construction and comprising a single longitudinal seal, in this case the top core wrap and the bottom core wrap layers are made of the same web material.

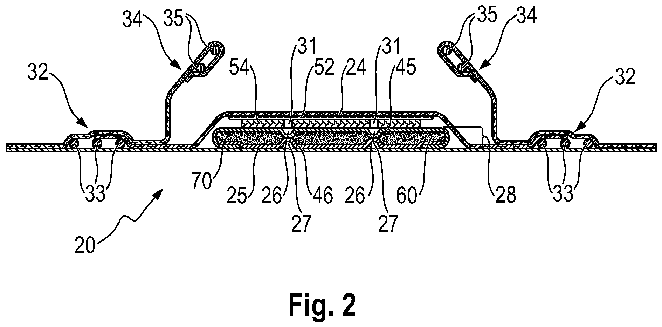

The core wrap layers may be bonded face to face but other bonding configurations are possible, in particular a C-wrap configuration where one of the top or bottom core wrap layer is larger than the other, so that flaps can be folded around the absorbent material and attached to the other core wrap layer, as illustrated in Fig. 2.

The total amount of SAP present in the absorbent core is adapted to the need of the expected wearer of the article. Diapers for newborns require less SAP than infant or adult heavy incontinence diapers. The amount of SAP in the core may be for example comprised from about 2 g to 50 g, in particular from 5 g to 40 g for typical baby diapers. The average SAP basis weight within the absorbent core may be for example of at least 50, 100, 200, 300, 400, 500 g/m2 or more, or from 200 g/m² to 400 g/m². The average SAP basis weight is the total amount of SAP in the core divided by the area delimited by the periphery of the SAP layer (including any channels).

### Channel(s)

The absorbent core 28 comprises a pair of elongated, absorbent material-free channels 26 symmetrically disposed on each side of the longitudinal centerline 80'. A channel is an area within the absorbent material layer 60 which is substantially free of absorbent material, so that the core wrap top layer 45 and core wrap bottom layer 46 can be directly bonded to each other through at least a portion of the length of the channel. Substantially free means there is no superabsorbent particles in the channels apart from particles that may be deposited accidentally in the channels during the core-making process. The first and second channels may have any shape. The invention is generally applicable to various channel shapes that have been suggested in the art. The first and second channels may be disconnected, as illustrated in Fig. 1. The first and second channels may also be connected, for example at one or both their extremities to form a U or O shape. Examples of channel shapes are disclosed in further details in WO2012170778A1, WO2012170781 (Kreuzer et al.). The absorbent core may also comprise additional channels, such as shorter front channels as is known in the art. The first and second channels preferably do not extend to any of the side of the absorbent layer, and thus each of the channel is surrounded by the absorbent material in the x, y plane.

The first and second absorbent channels are elongated in the longitudinal direction, having a length L' which is at least 20% of the length of the absorbent article. Unless otherwise mentioned, all the length reported herein are measured in the longitudinal direction y (i.e. length means the length as measured projected on the longitudinal centerline). The length L' of the elongated channel may be of from 20% and 80%, or from 20% to 70%, or from 30% to 60%, of the length L of the absorbent article. The first and second channels may have an entire length L' of at least 10 cm for typical absorbent cores (all lengths being measured in the longitudinal direction unless otherwise stated). The width of the first and second channel is typically at least 6 mm, preferably in the range of from 8 mm to 16 mm for at least the portion 68 of the channels that has an asymmetric bond. The bond 27 may typically have a width (Wb) that ranges from 25% to 50% of the channel width (Wc).

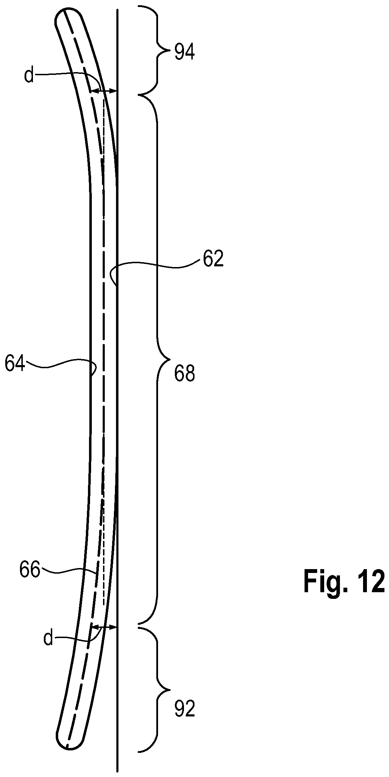

As represented on Fig. 12 for the left channel, each of the first and second channels comprise an inner edge 62, which is oriented towards the longitudinal axis 80' of the core, and an outer edge 64 which is oriented towards the closest longitudinal edge 284, 286 of the absorbent core. Each of the channels also comprised a channel centerline 66, which is disposed equidistantly between the inner edge 62 and the outer edge 64 of the channel 26. The first and second channels each preferably comprises a straight portion 68, which is substantially parallel (and thus also substantially straight) to the longitudinal centerline of the core 80'/article 80. This straight portion 68 of the channel may advantageously correspond with the portion of the channels that is provided with an asymmetrical bond according to the invention. The word "at least a portion" also encompasses the possibility that the whole of the channels are substantially parallel with the longitudinal centerline as indicated. Thus the first and second channels may also be substantially parallel with the longitudinal centerline over their entire length.

When the channel is not entirely straight and parallel with the longitudinal centerline, the portion 68 of each channel that is considered substantially straight and aligned with the longitudinal centerline 80' extends to the parts of channel whose central centerline do not deviate by a distance d of more than 2 mm from the portion of the channel that is straight and parallel with the longitudinal centerline. This is illustrated in Fig. 12 for the exemplary channel shape illustrated in the Figures, which comprises a main central portion 68 of the channel that is substantially parallel with the longitudinal centerline, as well as a curved front portion 92 and back portion 94. The curved front portion and curved back portion may be symmetrical or may differ, in the example shown the curved back portion 94 is shorter and more curved than the front curved portion 92.

The elongated absorbent material-free channel have a front end, a back end and a middle point. The front end and the back end of the channel are respectively the front extremity and the back extremity of the channel. By "front" and "back", it is meant closer to the front edge of the article and the back edge of the article respectively.

The middle point of the channel is the point disposed longitudinally equidistantly between the front end and the back end of the channel. The middle point is preferably present in the crotch region 37 of the article, so that the channel is generally centered on the crotch region 37 of the article.

### Asymmetric channel bond

The prior art discloses various absorbent cores with absorbent material free channels comprising a channel bond between the core wrap top layer and the core wrap bottom layer. Contrary to the prior art, the channel bond 27 of the invention is asymmetrical relative to the channel width for at least a portion of the first and second channels in the crotch region of the article, so that the channel width preferentially shrinks on the half of the channel width where the channel bond is weaker thus increasing the volume available for the absorbent material to swell in on that half of the channel.

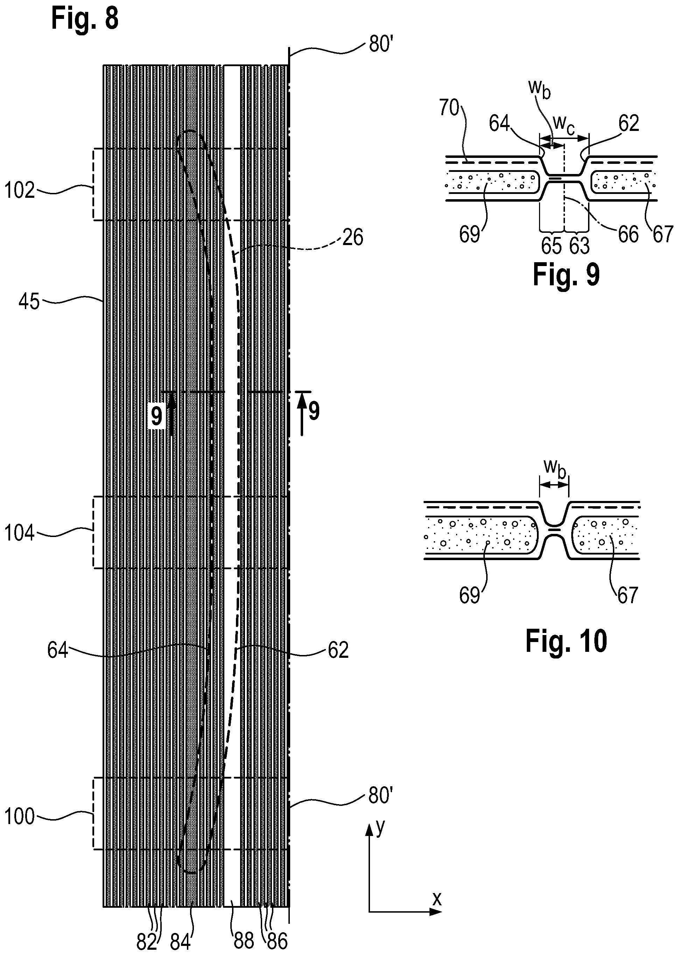

This is schematically illustrated in Fig. 9. In this schematic representation of a channel bond according to the invention, the channel bond is formed by an auxiliary glue 70 disposed on the inner side of one of the core wrap layer, in this example the core wrap top layer. The auxiliary glue 70 is disposed within the channel adjacent the outer edge 64 of the channel but is not present adjacent the inner edge 62 of the channel. More generally, the bond is stronger in the half 65 of the channel that extend from the outer edge to the channel centerline, referred herein as the outer half of the channel, compared to the opposite half that extends from the channel centerline to the inner edge of the channel, referred herein as inner half 63 of the channel. The bond has a width (Wb) that may typically range from 10% to 60% of the channel width (Wc), preferably from 25% to 50% of the channel width.

The channel bond may be adhesively formed. The channel bond may comprise or consist of longitudinally-extending auxiliary glue stripes 70. The auxiliary glue serves the dual purpose of immobilizing the absorbent material in the absorbent layer area (when dry) and forming a channel bond in the channel area. When the first and second channels each comprises at least a portion substantially parallel with the longitudinal centerline, the auxiliary stripes may be placed so as to be adjacent the inner edge or the outer edge of the channels in that portion, so that a strong channel bond is formed of the half of channel as desired, while the bond is absent or much weaker on the other half of the channel. In the example represented, it is desired that the core wrap preferentially delaminates on the inner edge of the channels, so that the absorbent material in the central absorbent area can more easily expand when the absorbent material swells in that area. In this example, the width of the channel shrinks preferentially on the inner half 63 of the channels that is free of absorbent material, releasing volume for the central absorbent area to expand into. On the outer half of the channel 65, on the other hand, the channel bond at least initially remains present when the absorbent core absorbs a liquid, as illustrated in Fig. 10, thus keeping the channel function of fluid transport in the longitudinal direction and core shape integrity. The remaining channel bond 37 thus provides structural integrity of the channel in the dry state and at least partially in the wet state. When the surrounding absorbent material swells during use, the channel can at least partially form a three-dimensional ditch along the bonded area of the channel.

The channel bond 27 may be at least partially formed by an auxiliary glue 70 deposited on at least one of the inner surface of core wrap top layer or the inner surface of the core wrap bottom layer in the channel. While adhesive bonding is preferred as it allows to easily control the channel bond strength without the risk of damaging the core wrap in the channel area, other bonding technology may be used, in particular fusion bond and ultrasonic bonding, that may be used to attach the core wrap top layer and core wrap bottom layer in one half of the channel.

The following is a simplified process for making an absorbent core as represented in the Figures. Of course, the process can be adapted to form other channel shape or using other bonding means as indicated above. The process of making a core and forming the core wrap bond as indicated above may for example comprise the steps of:
- providing a first core wrap layer, for example the core wrap top layer;
- applying a first auxiliary glue on the first core wrap layer, in a series of longitudinally extending stripes having a predetermined pattern, wherein the first auxiliary glue comprises a plurality of parallel, longitudinally-oriented auxiliary glue stripes, wherein two adjacent stripes are separated by a gap;
- depositing a first absorbent material on the first core wrap layer to form a first absorbent layer, wherein the absorbent material is at least partially immobilized on the first core wrap layer by the first auxiliary glue, and the first absorbent layer comprises at least a pair of elongated first and second channels having a portion that is substantially straight and parallel with the longitudinal centerline,
wherein the first auxiliary glue and the first absorbent material is applied so that the auxiliary glue is asymmetrically present in at least a portion of the first and second channels, in the exemplified cores wherein the auxiliary glue is present on the half of the channel adjacent to the outer edge 64 of the channel (outer half 65), and absent or at least less present, on the other half of the channel adjacent to the inner edge 62 of the channel (inner half 63),
- providing a second core wrap layer, which may be the core wrap bottom layer;
- combining the first core wrap layer with the first absorbent layer deposited thereon with the second core wrap layer to form an absorbent core according to the invention.

The absorbent core so made has a channel bond in half of the channel which is stronger than in the other half of the channel where the channel bond is weaker. The channel width thus preferentially shrinks from the inner edge or the outer edge of the channel, as desired, increasing the volume available for the absorbent material to swell in from the central absorbent area or lateral absorbent areas, as desired.

Optionally a second auxiliary glue and/or a second absorbent layer may also be applied on the second core wrap layer. The first and second auxiliary glue may each have a predetermined pattern as described herein, or it may be that the combination of the first and the second auxiliary glue form and auxiliary glue pattern of the invention. This optional choice may be more costly because as second glue applicator may be needed but a better absorbent material immobilization may be provided as the auxiliary glue is applied from both sides of the absorbent material layer. In any case, the auxiliary glue pattern obtained is irregular pattern, as will be described in further details below.

Of course other steps may be present in the process of making the absorbent core. For example, for airfelt-free cores made using SAP printing technology, the absorbent material is typically applied as one half layer on each core wrap substrate and comprise an additional microfibrous net to immobilize each half layer of absorbent material, as described above. An airfelt core (comprising a mix of cellulose fibers and superabsorbent particles) on the other hand may be sufficiently immobilized by one auxiliary glue layer, whereas the combination of a first and second auxiliary glue layers is also possible.

Typically, hotmelt adhesives are used in core making process. Hotmelt adhesives are solid at room temperature, thus they are typically heated to the molten state when they are applied on a substrate, before the resolidify, which is the so-called open time, a bond can be formed by pressing another substrate with the glue on the first substrate. Auxiliary glue may be applied in a series of stripes by contact applicators (slot glue) or alternatively non-contact applicator such as spiral or spray applicators. If the absorbent material is deposited on the core wrap substrate using SAP printing process as disclosed above, a thin coating of microfibrous glue is typically used to further immobilize the SAP particles on the substrate on which they are applied. This thin coating of microfibrous glue may be used to provide some integrity of the channel in the non-bonded half of the channel, but to at much less extent than the auxiliary glue.

### Auxiliary glue 70

As indicated previously, the absorbent core may comprise an auxiliary glue 70 disposed between the absorbent material and at least one of the core wrap top layer or bottom layer. In addition to its basic function of at least partially immobilizing the absorbent material on the core wrap top layer or bottom layer, the auxiliary glue may also be used to form the asymmetric bond pattern of the present invention.

The auxiliary glue 70 comprises a plurality of parallel stripes separated by gaps, as generally illustrated in Figs. 7-8. The stripes are typically longitudinally oriented. The auxiliary glue is at least present in the core channel area 74, which is the area of the core longitudinally delimited by the first and second channels. The auxiliary glue may also be present in the core front region 72, which is the area of the core disposed in the front of the channel, and optionally on the core back region 76 which is the area of the core disposed in the back of the channel. As shown in the example of Fig. 7, it may be chosen not to have auxiliary glue in the core back region 76 as this area of the core may comprise relatively less SAP than the rest of the core and thus require less immobilization.

The auxiliary glue 70 may extend transversally across the whole width W1 of the absorbent layer or may be shorter transversally than the absorbent layer. The width of the auxiliary glue 70 may be at least 50% the width of the absorbent layer, so that at least a significant proportion of the absorbent material is at least partially immobilized by the auxiliary glue. However the auxiliary glue does not necessarily extend transversally up to the longitudinal edges 604, 606 of the absorbent layer.

The auxiliary glue 70 is applied on at least one of the core wrap top layer or bottom layer and at least partially immobilizes the absorbent material deposited thereon. This absorbent material immobilization is particularly relevant to provide integrity of the absorbent material placement in the core wrap before the article is used. The auxiliary glue may be applied on the inner side of either the core wrap top layer and/or core wrap bottom layer, typically by slot glue application.

The auxiliary glue is present in the core wrap at least in the channel area 74 and at least partially forms the channel bond 27 between the core wrap top layer and core wrap bottom layer. By applying pressure in the channel area 74 during the core making process, the two layers of the core wrap are contacted through the channels and the adhesive provides an adhesive bond between these two layers through the channel.

Typically, auxiliary glues are hotmelt adhesives that are applied in core making process on at least one of the core wrap layer as substrate. Hotmelt adhesives are solid at room temperature, thus they are typically heated to the molten state when they are applied on a substrate. The time on the substrate before they resolidify is called open time. During the open time, which is very brief, the absorbent material in contact with the substrate may be immobilized by the auxiliary glue, and a bond may be formed with another substrate especially a channel bond but also the core end seal bonds or the core longitudinal side bonds. Exemplary patent disclosures of such adhesive bonding processes can be found for an airfelt or airfelt-free absorbent cores in WO2012/170798Al (Jackels et al.), EP2,905,000 (Jackels et al.) and EP2,905,001 (Armstrong-Ostle et al.). This auxiliary glue 70 may be applied on the core wrap top layer 45, or the core wrap bottom layer 46, or even both layers.

Conventional auxiliary glue pattern comprises a regular series of longitudinal stripes having uniform width and gap between stripes (typically 1 mm). However such a regular pattern cannot provide an asymmetric channel bond as claimed in the present invention. An irregular auxiliary glue pattern 70 that may be used to form the channel bond 27 according to the invention is illustrated in Fig. 8. This example comprises auxiliary glue longitudinal stripes 82, 84 in a non-conventional, irregular pattern. This adhesive bond pattern according to the invention varies the width of the stripes and/or the width of gaps between adjacent stripes so that the desired asymmetric bond strength is reliably achieved, also in a highspeed manufacturing context. Having an irregular pattern allows to vary the amount of auxiliary glue deposited in transversal direction (and optionally also in the longitudinal direction). The shape of the channel 26 is shown in dotted lines in Fig. 8.

As can be in Fig. 8, a majority of the auxiliary glue stripes overlapping the absorbent material layer outside the channels may be conventional stripes 82 separated by conventional gaps 86, e.g. each being 1 mm wide. According to this example of the invention, the auxiliary glue pattern further comprises a second gap 88 having a second gap width, wherein the second gap width 88 is larger than the first gap width 86, for example the second gap width may be three times or more, larger than the first gap width 86. Thus for portion of the channel 68 that is substantially aligned with the longitudinal centerline 80', this larger, second gap 88 is designed to be within the channel and adjacent the inner edge 62 of the channel so that the inner half 63 of the channel does not comprise auxiliary glue. Accordingly, there is no auxiliary glue bond and no or only a weak bond (if a microfibrous glue is present) in this inner half of the channel in that portion 68 of the channel. The outer half 65 of the channel on the other hand in that portion 68 of the channel comprises at least one auxiliary glue stripe. In the outer half of the channel as represented, two stripes of ordinary width of 1 mm separated by a 1 mm gap are present.

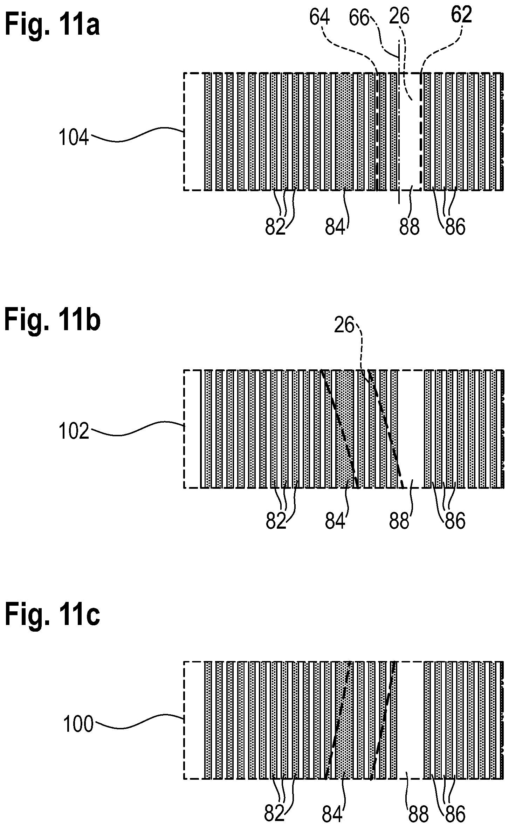

The asymmetric channel bond thus obtained can be generally present over the whole length of the portion 68 of the channel that is generally straight and parallel with the longitudinal centerline 80'. If the first and second channels are substantially parallel with the longitudinal centerline along their whole length, the auxiliary glue pattern can be very simple, comprised of standard stripes 82 separated by standard gaps 86, and provided with a single large gap 88 adjacent the edge of the channel where a weak bond, or no bond, is desired, in our example the inner edge of the channel. The channel half having the stronger bond may be provided by one or more standard stripe 82 or even a wider stripe 84. This is shown in a close-up view 104 on Fig. 11a for the middle of the straight portion 69 of the channel that is substantially parallel to the longitudinal centerline.

The auxiliary glue pattern as represented in Fig. 8 is more complex as the channels comprise front and back curved portions 92, 94. It may be desired that for these curved, end portions that a non-asymmetric, relatively strong bond pattern may be present. This can be obtained by providing at least one auxiliary glue stripe 84 having a second width, wherein the second width is larger than the first width of the stripes 82. The second width of the second stripe 84 may be at least twice, or at least three times, larger than the first width of the first stripes 82.The transversal gap, or distance, between the stripes may also be varied to adjust the amount of glue in each channel portions considered. This larger glue stripe 84 may be used to form a relatively strong channel bond in a curved front and/or back portion of the channel adjacent the front and/or back of the channel. Thus, as illustrated in Fig. 8, if desired, a strong bond can be provided at the front and/or back portion of the channel by disposing the larger glue stripe 84 to intersect with the front and/or back portion of the channel 26 so that a larger amount of glue per channel area is provided in these portions 100, 102 (see close-up view on Fig. 11b,c).

It is also to be noted that the bond strength may be disproportionally increased with the stripe width due to a better uniformity of the glue in larger stripes, for example it was noticed that a pair of 1 mm stripe separated by a 1 mm gap typically provides a weaker bond than a 2 mm wide stripe.

Using an auxiliary glue 70 pattern which comprises a series of longitudinal glue stripes 82, 84, wherein the stripes have different width and/or gap to vary the channel bond strength along the length of the channel was found to be simple and cost-efficient. Regularly disposed longitudinal glue stripes applied from a shim are a standard glue application pattern typically used for auxiliary glue adhesive. Thus, the channel bond design may be obtained simply by adjusting the shim pattern, from having a regular width and gaps to an irregular successions of width and/or gaps adapted to the channel design considered.

### Asymmetric Channel Bond Strength

The absorbent articles and absorbent cores of the invention are designed so that the channel bond is asymmetric relative to the channel width (Wc) for at least a portion of the first and second channels, so that, during use, the channel width preferentially shrinks on a pre-determined half of the channel thus increasing the volume available for the absorbent material to swell in that half of the channel. The preferential shrinking can be visually observed during a dunk test for example or a more sophisticated micro-CT scan of the channel in the wet state (see example below). The strength of the bond between the core wrap top layer and the core wrap bottom layer in each half of the channel can also be measured for a representative sample of the channel using the Static Peel Time Test as described below. In this case, the channel half e.g. in the inner half 63, that preferentially shrinks may typically have a lower Static Peel Time than the other channel half, e.g. the outer half 65, as measured according to the Static Peel Time Test disclosed herein. The bond strength in each half of the channel is measured in minutes according to the Static Peel Force Test disclosed hereinbelow. In this test the bond strength is measured on a 2.52 cm cutout in the central portion of the channel as disclosed further below.

Preferably the Static Peel Time of the preferentially shrinking channel half ranges from 0 min to 5 min and the Static Peel Time of the other channel half is at least 10 min, preferably ranges from 10 min to 120 min.

### Packages

The absorbent articles of the present disclosure may be placed into packages. The packages may comprise polymeric films, paper, and/or other materials. Graphics and/or indicia relating to properties of the absorbent articles may be formed on, printed on, positioned on, and/or placed on outer portions of the packages. Each package may comprise a plurality of absorbent articles. A ready-for-sale package preferably comprises a plurality of absorbent articles, wherein at least 50% of the articles in the package are according to the invention disclosed therein, preferably from 60 % to 100 % of the articles.

The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages. Accordingly, packages of the absorbent articles of the present disclosure may have an In-Bag Stack Height of less than about 120 mm, less than about 100 mm, less than about 80 mm, less than about 60 mm, but greater than about 20 mm, specifically reciting all 1mm increments within the specified ranges and all ranges formed therein or thereby, according to the In-Bag Stack Height test described herein. Alternatively, packages of the absorbent articles of the present disclosure may have an In-Bag Stack Height of from about 50 mm to about 120 mm, about 60 mm to about 120 mm, from about 70 mm to about 120 mm, or about 70 mm to about 100 mm, specifically reciting all 1 mm increments within the specified ranges and all ranges formed therein or thereby, according to the In-Bag Stack Height test described herein. The packages may comprise polymeric films comprising recycled material, such as about 20% to about 100%, about 30% to about 90%, about 30% to about 80%, about 40% to about 60%, or about 50% recycled material. The recycled material may comprise post-industrial recycled material (PIR) and/or post-consumer recycled material (PCR). In some instances, the polymeric films used for the packages may comprise two outer layers and one or more inner layers. The one or more inner layers may comprise the recycled material or may comprise more recycled material than the outer layers. The recycled material may comprise recycled polyethylene. The recycled material may comprise recycled polyethylene PIR from trim from the packaging operation.

The package material may comprise paper, paper based material, paper with one or more barrier layers, or a paper/film laminate. The package material may be in the range of about 50 gsm to about 100 gsm or about 70 gsm to about 90 gsm and the one or more barrier layers may be in the range of about 3 gsm to about 15 gsm. The paper based package material with or without one or more barrier layers may exhibit a machine direction tensile strength of at least 5.0 kN/m, a machine direction stretch of at least 3 percent, a cross-machine direction tensile strength of at least 3 kN/m, and a cross-direction stretch at break of at least 4 percent, each as determined via ISO 1924-3.

The paper based package material or paper based package material comprising a barrier layer or film may be recyclable or recyclable in normal paper recycling operations. The recyclability extent of the paper based package may be determined via recyclable percentage. The paper based package of the present disclosure may exhibit recyclable percentages of 70 percent or greater, 80 percent or greater, or 90 percent or greater. The paper based package of the present disclosure may have a recyclable percentage of between 70 percent to about 99.9 percent, between about 80 percent to about 99.9 percent, or between about 90 percent to about 99.9 percent. In one example, the package material of the present disclosure may exhibit a recyclable percentage of from about 95 percent to about 99.9 percent, from about 97 percent to about 99.9 percent, or from about 98 percent to about 99.9 percent. The recyclable percentage of the paper based package may be determined via test PTS-RH:021/97 (Draft Oct. 2019) under category II, as performed by Papiertechnische Stiftung located at Pirnaer Strasse 37, 01809 Heidenau, Germany. In another instance, the paper based packages of the present disclosure may exhibit an overall "pass" test outcome as determined by PTS-RH:021/97 (Draft Oct. 2019) under category II method. Any of the paper based packages may have opening features, such as lines of perforation, and may also have handles.

### Examples

The present invention is applicable to a wide range of absorbent cores and absorbent articles incorporating these cores. The dimensions and materials used may vary depending on the intended applications as is known in the art. For example, absorbent cores for larger size diapers will typically be longer and larger and comprise more absorbent material than absorbent cores for smaller size diapers.

The following describe size 4 and size 5 core examples that were made to illustrate dimensions and materials of the invention. The absorbent cores comprised a pair of core channels and are as generally represented in the Figures. These absorbent cores may be used in an absorbent article, e.g. taped diaper or pant diaper. The article may optionally comprise a pair of distribution channels in a distribution layer as illustrated in Figs. 2-3. The absorbent cores described below may be for example used in a "size 4" and a "size 5" pant diaper, which can be recommended ( as indicated on the commercial packaging) for baby weighing between 9 kg to 15 kg, and 12 k to 17 kg respectively. For both sizes exemplified, the absorbent core may be incorporated in a taped or pant diaper comprising a dual acquisition-distribution system comprising a latex bonded, nonwoven acquisition layer 52 and a cross-linked cellulose fibers distribution layer 54 between the acquisition layer and the absorbent core, having a shape as generally illustrated in Figs. 1-5 as described above. The width of the rectangular acquisition layer 52 is 95 mm and the maximum width of the distribution layer 54 in this example is 85 mm.

The key dimensions of the cores are reported in Table 1 below. All widths are measured parallel to the transversal direction (x) and all lengths are measured parallel to the longitudinal direction (y).

**Table 1: dimensions of the exemplary cores**

| *All distances in mm* | Size 4 | Size 5 |
|---|---|---|
| L (length of the article) | 480 | 505 |
| L' (length of the absorbent layer) | 340 | 400 |
| W1 (width of the absorbent layer) | 110 | 110 |
| Length of the core (including core wrap) | 370 | 430 |
| Width of the core (including core wrap) | 120 | 120 |
| L" (length of the channel 26) | 197 | 218 |
| Wc (Width of channel 26) | 8 | 8 |
| Straight portion of the channel 68 | 112 | 135 |

The core wrap comprised a top layer C-wrapped around a bottom layer (as in Fig. 2) with adhesive longitudinal seals. The top layer had a width of 165 mm, the bottom layer has a width of 130 mm. The width of the core bag was approximately 120 mm. The top layer was an 8 gsm water permeable propylene SMS nonwoven and the bottom layer is a hydrophobic, 10 gsm propylene SMS nonwoven. The core wrap was sealed by gluing at its front edge and its back edge. The absorbent material consisted entirely of SAP particles, which are deposited for one half on the top layer and the other half on the bottom layer of the core wrap and immobilized by a microfibrous glue (SAP printing method as described above). An auxiliary slot glue layer 70 was disposed on the inner side of the core wrap top layer with an overall width of 81 mm and a pattern of alternating stripes as represented in Fig. 8 (small stripes 82 are 1 mm wide, the large stripe 84 is 3 mm wide, the small gap 86 are 1 mm wide and large gap 88 is 5 mm wide). The total amount of SAP in the core was 13.5 g in the size 4, and 13.9 g in size 5. The absorbent layer had a generally rectangular shape. The basis weight of the SAP in the absorbent layer was longitudinally profiled so that more absorbency is available towards the middle and the front of the core. The total length of the core wrap was about 15 mm longer in the front and in the back than the length of the absorbent material layer to provide for front and back core wrap end seals 280', 282'.

**Table 2: SAP distributions of the exemplary cores**

| *Amount of SAP in g* | Size 4 | Size 5 |
|---|---|---|
| Core front region 72 | 1.84 | 2.14 |
| Core back region 74 | 2.23 | 2.03 |
| Central absorbent area 67 (between the channels) | 3.99 | 3,77 |
| Lateral channel area 69 (both areas together) | 5.44 | 5.96 |
| Total | 13.5 | 13.9 |

Of course these values are the target value, with the real values may diverge +/- 5% due to tolerance in a high-speed manufacturing process.

The distance between the inner edge of the first and second channels was 30 mm, the distance between the outer edge of each of the channel and the longitudinal edge of the absorbent layer was 37 mm. Based on these, the swelling pressure ratio between the central absorbent area and the lateral absorbent area was calculated to be 1.49.

A comparative core was provided having the same characteristics with the difference that the auxiliary glue pattern was regular, comprising alternating 1 mm wide glue stripes separated by 1 mm wide gaps.

### Static Peel Time Test:

The channel bond strength for the inner half and outer half of the left and right channels was measured for the Size 5 core examples disclosed above according to the Static Peel Time Test described below. The core laminate samples were cut in middle of the central straight portion 68 of the channel The measurements are indicated in Table 3 below for the inventive example and Table 4 for the comparative example.

**Table 3: Static Peel Time (min) - Inventive Example**

| | Left Channel Outer half | Left Channel Inner half | Right Channel Outer half | Right Channel Inner half |
|---|---|---|---|---|
| | 20.3 | 0.0 | 15.6 | 0.0 |
| | 11.8 | 0.2 | 53.5 | 0.0 |
| | 27.0 | 0.0 | 131.8 | 0.0 |
| | 41.1 | 0.0 | 10.6 | 0.5 |
| | 50.4 | 0.1 | 36.0 | 0.0 |
| Average | 30.1 | 0.1 | 49.5 | 0.1 |

**Table 4: Static Peel Time (min) - Comparative example**

| | Left Channel Outer half | Left Channel Inner half | Right Channel Outer half | Right Channel Inner half |
|---|---|---|---|---|
| | 9.8 | 22.6 | 2.1 | 11.9 |
| | 37.6 | 0.6 | 29.6 | 27.3 |
| | 0.5 | 122.0 | 50.9 | 11.9 |
| | 34.0 | 24.7 | 53.0 | 5.0 |
| | 10.0 | 2.9 | 3.4 | 0.0 |
| Average | 18.4 | 34.6 | 27.8 | 11.2 |

As can be seen from Table 3 and Table 4, the inventive example had clearly asymmetric channels as demonstrated by the consistent differences in Static Peel Time between the inner half and the outer half of the channel. The comparative example on the other hand had highly variable Static Peel Time between the inner and outer halves of channel.

### Dunk Test

The inventive and comparative cores were submitted to a Dunk Test, in which the Size 5 core samples were completely covered by a saline solution for 5 min. The core was then kept vertical for 30 s after the 5 min dunk to let the liquid drool out of the core. The amount of fluid acquired in g was divided by the amount of SAP in g to calculate the total capacity used by each core. The test was repeated 6 times and the average values are reported in Table 4 below.

**Table 5: Dunk Test**

| | Total capacity (g/g) | |
|---|---|---|
| | Average | Std dev |
| Comparative Example | 29.04 | 0.18 |
| Inventive Example | 29.42 | 0.05 |

### Micro-CT

The absorbent cores (for finished product, carefully remove the other parts of the diaper and leave only the core) with the top side up were placed in a plastic dish and loaded with 250 ml of 0.9% Saline (± 1%), with a graduated cylinder. After 5 minutes (± 30 seconds) waiting time, if any liquid in the dish at the sides of the core which has not been in contact with the core, then the plastic or metal dish was held slanted in different directions, to allow any free liquid to be absorbed. Wait another 5 minutes (± 30 seconds) to allow all saline to be absorbed. Some drops may retain in the plastic or metal dish.

Loaded samples were mounted flat on an acrylic plate (thickness 10 mm) and scanned In a comouter tomograph (Zeiss Metrotom 1500 G3). Voxel size was adjusted to 102 µm (scanning of entire sample), all scanning parameter and reconstruction work set to automatic mode.

The micro-CT apparatus can take pictures of a cross-section of the swollen absorbent core. The micro-CT showed that in the inventive examples the inner half of the channel delaminated while in the outer half of the channel, the core wrap top layer and the core wrap bottom layer remained bonded.

### Test methods

The values indicated herein are measured according to the methods indicated herein below, unless specified otherwise. All measurements are performed at 22°C ± 2°C and 50% ± 5% RH, and samples should be kept at least 24 hours outside of the bag in these conditions to equilibrate before conducting the tests, unless indicated otherwise.

### Swelling pressure comparison

The asymmetric channel may be designed so that the side of the channels that has the lower or no bond strength is adjacent the edge of the channel having the higher swelling pressure. In the central portion of the channel that is substantially straight and aligned with the longitudinal centerline, the swelling pressure in the central absorbent area can be easily compared with the swelling pressure in the lateral absorbent area by dividing the amount of superabsorbent particles in each zone by the square of the width of the zone. The swelling pressure in each area thus varies depending on the amount of superabsorbent particles in each area and the width of each area.

The width of the central zone is measured as the shortest distance between the inner edges of each channel, and the width of the lateral zone is shorter distance between the outer edge of the channel and the longitudinal edge of the absorbent layer.

### Static Peel Time Test

The Static Peel Time Test method is typically used to assess the strength of a channel bond. The method can also be used, as described below, to measure and compare the strength of the bond on the inner half of the channel vs. the outer half of the channel. The sample taken in the present method has a width, measured in the longitudinal direction, of 25.4 mm (1 inch). This test method measures in minutes how long the channel bond withstands a constantly applied vertical peel force of about 150 grams under standardized conditions (Static Peel Time).

### Equipment

| | |
|---|---|
| Medium Clip | Medium Binder Clips 25 mm Capacity (#72050). ACCO World Product. Other suppliers: Yihai Products (#Y10003), Universal Office Products (#10210), Diamond (#977114), or equivalent. The clip weights 4.5 g +/- 1 g. |
| Large Clip | Large Binder Clips 2 inch (50.8mm). ACCO World Product. Other suppliers: Yihai Products, Universal Office Products, Diamond, or equivalent |
| Test Stand | RT-10 room temperature (Shear Tester) w/ timer. ChemInstruments, 510 Commercial Drive, Fairfield Ohio 45014-9797, USA; or equivalent. Must be placed in a vibration free area. |
| Weight | 150g (+/- 1g) TW150 Shear Tester Weight with hook on top (to attach to the clip). ChemInstruments, 510 Commercial Drive, Fairfield Ohio 45014-9797, USA; or equivalent |
| Cutting Tools | Scissors and a 25.4 mm (1 inch) cutter (convenient source, e.g. JDC Precision Sample Cutter made by Thwings-Albert Instrument Company Philadelphia USA, cat# 99, cut width 25.4 mm, accuracy at least +/- 0.1 mm) |
| Metal Ruler | Traceable to NIST, DIN, JIS or other comparable National Standard, graduated in mm, longer than the length to be measured. |
| Temperature | Testo-temperature device (or equivalent) to measure temperature at sample height with an accuracy of ± 0.5 °C and ± 2.5 % RH in the range between -10°C and +50°C. Testo GmbH & Co., Postbox 1140, D-79849 Lenzkirch (www.testo.com) Article number for Testo 625: 0563 6251. |

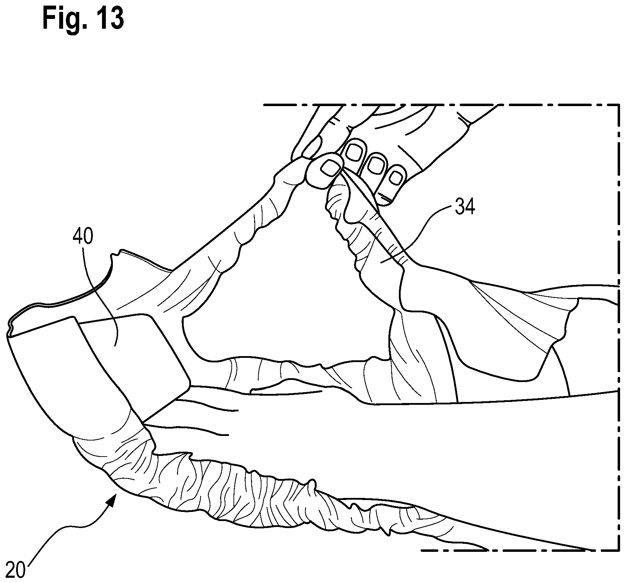

### Core preparation (see Fig. 13):

The absorbent articles are left at least one week in their closed package after production for the bond strength to stabilize. The absorbent core can be extracted from a commercial article (in the following a diaper, but the method applicable to other type of absorbent articles) as follows:
1. Open the diaper topsheet side up and place the diaper flat onto a table. A taped diaper can be held with one hand and the ears carefully removed. For pant diapers, the side seams are cut open and waistbands removed. Barrier cuffs are carefully removed along the cuffs continuous bond (outer edge) on both sides of the articles.
2. Gently remove topsheet and acquisition system without damaging the core end seals, if present. The backsheet does not need to be removed.

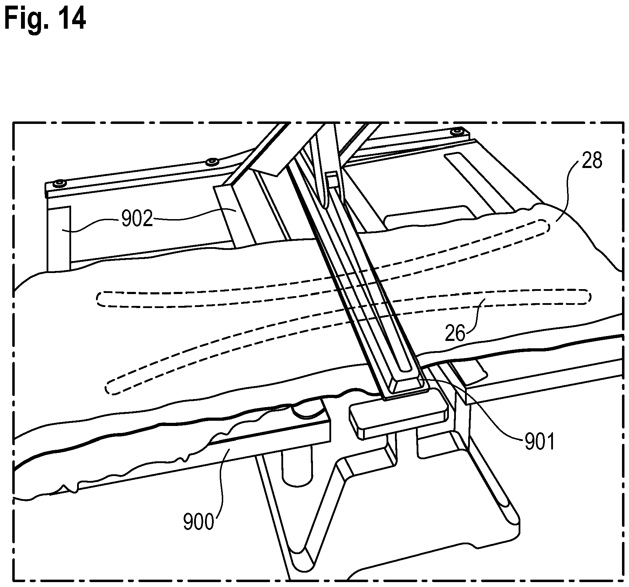

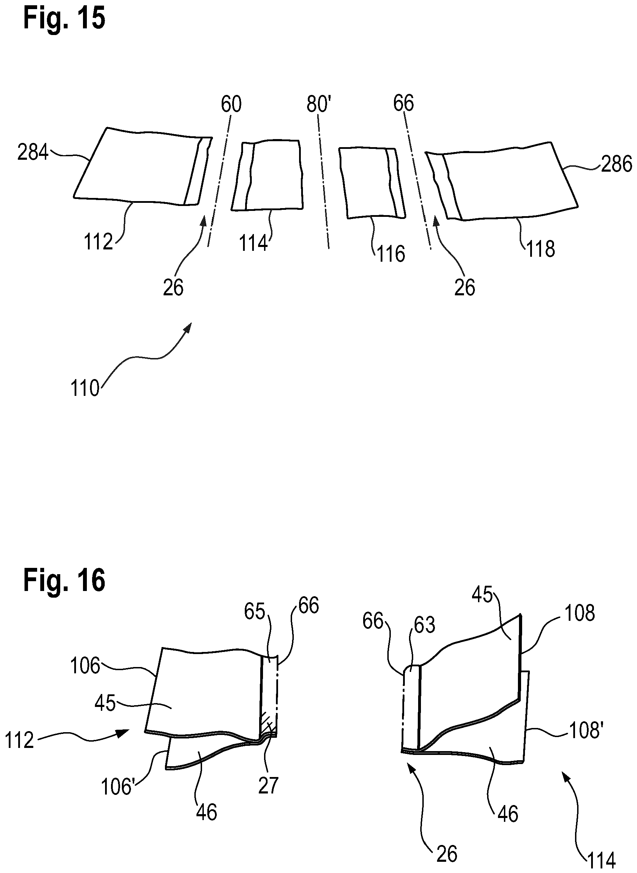

### Channel Sample preparation (see Figs. 14, 15):

For each channel proceeds as follows:
1. Put the core under a lamp table or a UV-light to identify the shape of the channel.
2. Mark using a ruler and pencil the region of the absorbent core to be tested by drawing a line perpendicular to the longitudinal centerline across the whole of the core. For example in the middle of the portion of the channel that is substantially aligned with the longitudinal centerline.
3. Lay the core (28) on a supporting table (900) fitted with the 25.4 mm wide cutter (901) and align to the transversal line drawn over the channels. Double sided tapes (902) may be used to keep the sample in place. The table comprises two grooves or gaps on each side of the area to be cut so that the cutter blade can penetrate through the thickness of the core.
4. Cut the core in transversal direction to obtain a band of core laminate (110) comprising the first and second channels 26 (target width of the band = 25.4 ± 2 mm).
5. The band of core laminate obtained comprises a first channel and a second channel per band. The band (110) is then cut with the scissors in its middle along the longitudinal centerline of the core, as well as through the centerline of the channels to isolate fours samples (112, 114, 116, 118) in total: an inner and outer samples for each of the left and right channels. Note for each sample to which channel and which side they correspond to (e.g. left or right channel, inner or outer half).
6. Gently remove any absorbent material outside the channel bond that is between the core wrap top layer (45) and the core wrap bottom layer (46). For this, it may be necessary to open any core wrap longitudinal side seals on the outer edges 284, 286 of the sample, if needed.
7. For each of the samples, the free edges 106, 106', 108, 108' of the core wrap layers 45, 46 opposite the channel half 63, 65 are clamped during the test. The clamped portion of the core wrap layers typically have a minimum length distance at least 10 mm for this purpose, 15-20 mm being ideal, to ensure a proper clamping of the core wrap material into the clamps.

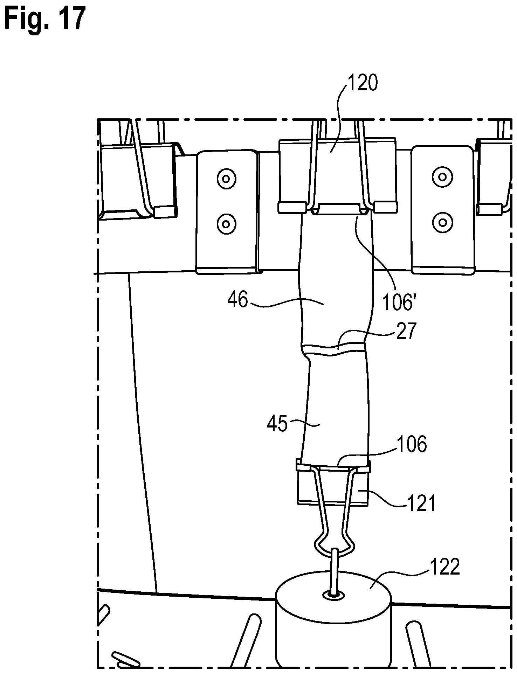

### Test Procedure (see Fig. 17):

Set up the tester in an area where the temperature is constant at 23°C and ensure that the tester have at least 2 h time to reach this temperature.
1. Clamp the outer edge (106') of the bottom layer (46) of the sample into the jaw of the large binder (120) clip hanging at the top of the tester bar.
2. Clamp the other binder clip (medium size) (121) to the top layer (45) of the core wrap at its outer edge (106). The channel edge 66 of the sample is facing away from the experimenter.
3. Gently attach the 150 g weight (122) to the medium binder clip and lower the clamped weight slowly until the weight hangs freely on the test sample. Avoid any sudden drop of the weight.
4. As soon as the weight is released, push the timer reset button for that sample to begin the timer at 0 minutes. NOTE: The timer must be checked to ensure that it has begun counting from 0.0 min. The operator should look for the number to change from 0.0 min to 0.1 min.
5. Repeat procedure above for each sample prepared. The Test Stand allows testing several samples in parallel.
6. The timers will stop once the sample weight has fallen on the bottom plate due to the bond breaking. The bottom plate comprises a switch linked to the timer so that it automatically stops when the weight has fallen down. The time recorded is the Static Peel Time for that sample (expressed in minutes). Note: if the weight falls down due to the sample slipping out of the clip (121) or due to core wrap tear without the bond breaking, the test needs to be rerun on a new sample.
7. If the sample weight has not fallen after 999 minutes, the Static Peel Time is reported to be 999 minutes (maximum Static Peel Time).
8. For a commercial article of a given construction, the measurements are repeated at least 5 times on equivalent articles (e.g. 5 randomly selected diapers in a commercially-sourced diaper package ) to obtain an average value of the Static Peel Time for a given channel section. The measurement is conducted for each half and each channel separately. The Static Peel Time are measured and reported for the inner and outer half of right and left channels separately.

### In Bag Stack Height Test

The in-bag stack height of a package of absorbent articles is determined as follows. The absorbent article packages are equilibrated at 23 +/- 2 deg C and 50 +/relative humidity prior to measurement. Place an unopened package directly on a rigid, horizontal surface such that stacks of folded absorbent articles are consecutively disposed on top of each in a direction perpendicular to the horizontal plane. Use a steel ruler or equivalent to measure the maximum distance between the horizontal surface and the most distal portion of the package from the horizonal surface. Three identical packages (same size packages and same absorbent article count per stack) are measured and the arithmetic mean is reported. The In-Bag Stack Height = (arithmetic mean/folded absorbent article count per stack) x 10 is calculated and reported within +/-0.5mm.

### Misc

As used herein, the terms "comprise(s)" and "comprising" are open-ended; each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essentially of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "preferably", "advantageously", "in particular" and the likes also qualify features which are not intended to limit the scope of the claims unless specifically indicated to do so.

Unless indicated otherwise, the description and claims refer to the absorbent core and article before use (i.e. dry, and not loaded with a fluid) and conditioned at least 24 hours at 23 °C +/- 2 °C and 50 +/- 5% Relative Humidity (RH).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A personal hygiene absorbent article (20) for absorbing fluid such as urine, the article comprising a front waist region (36), a crotch region (37), a back waist region (38), a longitudinal centerline (80) extending in a longitudinal direction (y), and a length L measured along the longitudinal centerline, wherein the crotch region (37) is centered on the middle of the longitudinal centerline and extends longitudinally over 50% of the length of the article;
the article comprising a topsheet (24), a backsheet (25) and an absorbent core (28) between the topsheet and the backsheet;
the absorbent core comprising a core wrap top layer (45), a core wrap bottom layer (46), an absorbent material layer (60) between the core wrap top layer and bottom layer, wherein the absorbent material comprises or consists of superabsorbent polymer particles optionally mixed with cellulose fibers and/or synthetic fibers;
wherein the absorbent material layer (60) comprises a front edge (600), a back edge (602) and two longitudinal edges (604, 606);
wherein the absorbent material layer comprises a first and second longitudinally-elongated, absorbent material-free channels (26), wherein the first channel and the second channel are respectively disposed symmetrically on each side of the longitudinal centerline (80), wherein the first channel and the second channel may be connected to another or alternatively may be unconnected to another, preferably wherein the first and second channels are at least partially present in the crotch region of the article,
wherein each channel has an inner edge (62) closer to the longitudinal centerline (80), an outer edge (64) closer to a longitudinal edge (604, 606) of the absorbent layer, a channel centerline (66) disposed at equal distance from the inner edge (62) and the outer edge (64) of the channel, a channel outer half (65) between the channel centerline (66) and the outer edge (64), an inner half (63) between the channel centerline (66) and the inner edge (62), and a width (Wc) as measured between the inner edge and the outer edge, wherein the width is constant or vary along the length of the channel;
wherein the absorbent layer comprises a central absorbent area (67) between the inner edges of the first and second channels, a first lateral absorbent area (69) between the outer edge (63) of the first channel (26) and the closest longitudinal edge (604) of the absorbent layer (60), a second lateral absorbent area (69) between the outer edge of the second channel (64) and the closest longitudinal edge (606) of the absorbent material layer (60);
wherein the first channel and the second channel each comprises a channel bond (27) between the core wrap top layer (45) and the core wrap bottom layer (46);
**characterized in that** the channel bond (27) is asymmetric relative to the channel width (Wc) for at least a portion (68) of the first and second channels (26), so that during use, the channel width preferentially shrinks on a pre-determined half (63) of the channel, thus increasing the volume available for the absorbent material to swell **in that** half (63) of the channel (26).

2. The absorbent article according to claim 1, wherein the channel bond (27) is weaker on the inner half (63) of the channel (26), so that the channel width (Wc) preferentially shrinks from the channel's inner edge (62) thus increasing the volume available for the absorbent material in the central absorbent area (67) to swell in.

3. The absorbent article according to any of the preceding claims, wherein the channel bond (27) comprises or consists of an adhesive bond (70), wherein the adhesive is asymmetrically disposed at least in the a portion in the channel (68) so that there is more glue in one half of the channel (65) than in the other half of the channel (63), in particular where there is more glue in the outer half (65) of the channel than in the inner half (63) of the channel.

4. The absorbent article according to any of the preceding claims, wherein at least a portion (68) of each of the first and second channels is substantially parallel with the longitudinal centerline (80, 80'), and the asymmetric bond (27) is at least present in said portion of the channel substantially parallel with the longitudinal centerline.

5. The absorbent article according to the preceding claim, wherein for the substantially parallel portion (68) of the channel, one area selected from the central absorbent area (67) and the lateral absorbent areas (69) has a higher calculated swelling pressure than the other area, wherein the calculated swelling pressure is proportional to the amount of absorbent material in the portion of the absorbent area considered divided by the width squared of the absorbent area, and the pre-determined half (63) of the channel that shrinks is the half of the channel closer to the absorbent area having the higher calculated swelling pressure.

6. The absorbent article according to the preceding claim, wherein the central absorbent area has a higher swelling pressure than any of the first and second lateral absorbent areas and the inner half (63) of the channel has a weaker bond than the outer half (65) of the channel, so that during use the channel width preferentially shrinks on the inner half of the channel thus increasing the volume available for the absorbent material in the central absorbent area (67) to swell in the inner half of the channel.

7. The absorbent article according to any of the preceding claims, wherein the absorbent material is partially immobilized within the core wrap by an auxiliary glue (70) disposed on at least one of the core wrap top layer (45) and core wrap bottom layer (46);
wherein the auxiliary glue comprises a plurality of longitudinally-oriented stripes (82, 84), wherein two adjacent stripes are separated by a gap (86, 88);
wherein the asymmetric channel bond (27) is at least partially formed by the auxiliary glue (70) being asymmetrically disposed in at least a portion of the channel, preferably the portion of the channel (68) which is substantially parallel with the longitudinal centerline (80').

8. The absorbent article according to preceding claim, wherein the auxiliary glue is present in one of the channel half (65) but not the other channel half (63) in said portion (69) of the channel, in particular wherein the auxiliary glue is present adjacent the outer edge (64) of channel but not the inner edge (62) of the channel (26).

9. The absorbent article according to any of the preceding claims, wherein each of the first and second channels (26) comprise at least a central portion (68) substantially parallel with the longitudinal centerline (80), and optionally a front portion (92) and/or a back portion (94) that is/are not parallel with the longitudinal centerline, in particular wherein the front and/or back portion of the channels is curved and/or is straight but not parallel to the longitudinal direction.

10. The absorbent article according to any of the preceding claims, wherein the channel half (63) that preferentially shrinks has a lower Static Peel Time than the other channel half (65) for at least the portion (68) of the first and second channels (26), as measured according to the Static Peel Time Test disclosed herein, preferably wherein the Static Peel Time of preferentially shrinking channel half ranges from 0 min to 5 min and the Static Peel Time of the other channel half is at least 10 min, preferably ranges from 10 min to 120 min.

11. The absorbent article according to any of the preceding claims, wherein the first and second channels each have an entire length (L") of at least 10 cm as measured parallel to the longitudinal direction (y).

12. The absorbent article according to any of the preceding claims, wherein the portion (68) of the first and second channels (26) having an asymmetric bond relative to the channel width (Wc) has a length of at least 5 cm as measured parallel to the longitudinal direction (y).

13. The absorbent article to any of the preceding claims, wherein the width of the first and second channel is at least 6 mm, preferably in the range of from 8 mm to 16 mm for at least the portion (68) of the channels that has an asymmetric bond, preferably wherein the bond (27) was a width (Wb) that ranges from 25% to 50% of the channel width (Wc).

14. A ready-for-sale package comprising a plurality of absorbent articles, wherein at least 50% of the articles in the package are according to any of the preceding claims, preferably from 60 % to 100 % of the articles are according to any of the preceding claims.

15. An absorbent core comprising a core wrap top layer (45), a core wrap bottom layer (46), an absorbent material layer (60) between the core wrap top layer and bottom layer, wherein the absorbent material comprises or consists of superabsorbent particles optionally mixed with cellulose fibers, and a longitudinal centerline (80'),
wherein the absorbent material layer (60) comprises a front edge (600), a back edge (602) and two longitudinal edges (604, 606),
wherein the absorbent material layer comprises a first and second longitudinally-elongated, absorbent material-free channels (26), wherein the first channel and the second channel are respectively disposed symmetrically on each side of the longitudinal axis (80'), wherein the first channel and the second channel may be connected to another or alternatively may be unconnected to another,
wherein each channel has an inner edge (62) closer to the longitudinal centerline (80'), an outer edge (64) closer to a longitudinal edge (604, 606) of the absorbent layer, a channel centerline (66) disposed at equal distance from the inner edge (62) and the outer edge (64) of the channel, a channel outer half (63) between the channel centerline (66) and the outer edge (63), an inner half (65) between the channel centerline (66) and the inner edge (62), and a width (Wc) as measured between the inner edge and the outer edge, wherein the width is constant or vary along the length of the channel;
wherein the absorbent layer comprises a central absorbent area (67) between the inner edges of the first and second channels, a first lateral absorbent area (69) between the outer edge (63) of the first channel (26) and the closest longitudinal edge (604) of the absorbent layer (60), a second lateral absorbent area (69) between the outer edge of the second channel (64) and the closest longitudinal edge (606) of the absorbent material layer (60);
wherein the first channel and the second channel each comprises a channel bond (27) between the core wrap top layer (45) and the core wrap bottom layer (46);
**characterized in that** the channel bond (27) is asymmetric relative to the channel width (Wc) for at least a portion (68) of the first and second channels (26), so that, during use, the channel width preferentially shrinks on a pre-determined half (63) of the channel thus increasing the volume available for the absorbent material to swell **in that** half (63) of the channel (26).
